# EUROPEAN PATENT APPLICATION

(11) **EP 4 070 799 A1**
(43) Date of publication of application: **12.10.2022**
(21) Application number: 21167329.8
(22) Date of filing: 08.04.2021
(51) Int. Cl.: A61K 31/706, A61K 31/7084, A61K 45/06, A61P 21/00, A61P 43/00

(54) **COMPOSITIONS FOR THE IMPROVEMENT OF SPORT PERFORMANCE**

(71) Applicant: Nuvamid SA, 1066 Epalinges (CH)
(72) Inventor: BERMOND, Guillaume, 13007 Marseille (FR); GARCON, Laurent, 13960 Sausset les Pins (FR)
(74) Representative: August Debouzy

(57) **Abstract**

The invention relates to compositions for the improvement of sport performance comprising compounds of formula I or formula Ia and at least one acceptable carrier.

## Description

### FIELD OF THE INVENTION

The invention relates to compositions and their use for the improvement of sport performance, such as the improvement of at least one parameter selected amongst the maximal heart rate, the ventilatory threshold 1, the ventilatory threshold 2, the VO2max and maximal aerobic power and their combinations.

### TECHNICAL BACKGROUND

When it comes to improving athletic or sport performances, whether it concerns professional or occasional athletes, the first thing that most people think of is focusing on functional exercises that translate directly into the sport. However, nutrition plays an important part in the improvement of physical or sport performance. More specifically, the organism needs a steady supply of macronutrients such as lipids, carbohydrates and proteins as well as micronutrients such as vitamins and minerals. However, it is not always feasible to ingest the sufficient amount of specific nutrients, such as amino-acids, vitamins or lipids, during the day with a conventional nutrition. Dietary compositions can be used to provide a specific amount of a specific nutrient or combinations of nutrients to the athlete. Provided herein are compounds, compositions, and kit of parts for the improvement of sport performance.

### SUMMARY OF THE INVENTION

These goals are achieved, at least in part, by the present invention.

The present invention relates to a compound of formula (I): 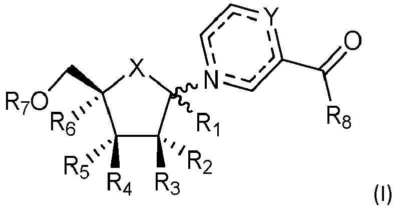 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 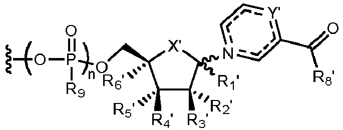 wherein n is an integer chosen amongst 1 or 3; wherein:
R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
R11 is selected from C1-C10 alkyl, C3-C10 cycloalkyl, C5-C18 aryl, C1-C10 alkylaryl, substituted C5-C12 aryl, C1-C10 heteroalkyl, C3-C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of Rlla is independently selected from H and (C1-C6) alkyl and Rllb is independently selected from OH, C1-C6 alkoxy, NH2, NH(C1-C6 alkyl) or N(C1-C6 alkyl)2 ;
R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
R13 and R14 are independently selected from H, C1-C8 alkyl and C1-C8 alkyl aryl;
Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
Y is selected from CH, CH2, C(CH3)2 and CCH3;
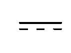 represents a single or double bond according to Y; and
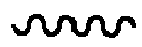 represents the alpha or beta anomer depending on the position of R1,
or a compound of formula (1a) 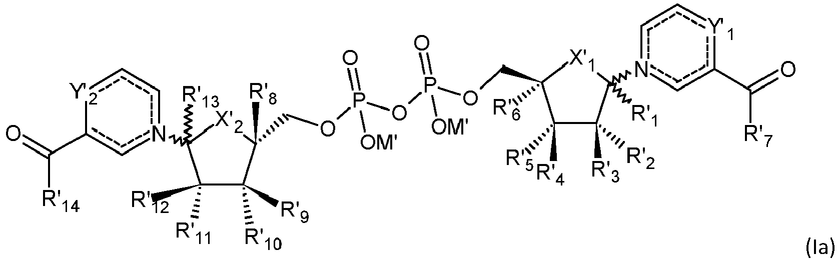 or pharmaceutically acceptable salts, hydrates, crystals, stereoisomers and/or solvates thereof, wherein:
   X'1 and X'2 are independently selected from O, CH2, S, Se, CHF, CF2 and C=CH2;
   R'1 and R'13 are independently selected from H, azido, cyano, C1-C8 alkyl, C1-C8 thio-alkyl, C1-C8 heteroalkyl and OR; wherein R is selected from H and C1-C8 alkyl;
   R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(0)NH(C1-C12)alkyl, C(O)O(C1-C12)alkyl, C(O)aryl, C(O)(C1-C12)alkyl aryl, C(O)NH(C1-Cl2)alkyl aryl, C(O)O(C1-C12)alkyl aryl or C(O)CHRAANH2, wherein RAA is a side chain selected from a proteinogenic amino acid ;
   R'6 and R'8 are independently selected from H, azido, cyano, C1-C8 alkyl and OR; wherein R is selected from H and C1-C8 alkyl;
   R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
   Y'1 and Y'2 are independently selected from CH, CH2, C(CH3)2 or CCH3;
   M' is selected from H or a suitable counter-ion;
   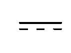 represents a single or a double bound depending on Y'1 and Y'2; and
   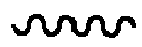 represents the alpha or beta anomer depending on the position of R'1 and R'13,
   and their combinations and at least one carrier, for use in the improvement of sport performance.

Advantageously, the compound according to the invention is selected from compounds I-A to I-J from Table 1 below ci-dessous or a pharmaceutically acceptable salt or solvate thereof:

**[Table 1]**

| **Composes (anomères)** | **Structure** |
|---|---|
| I-A (beta) | 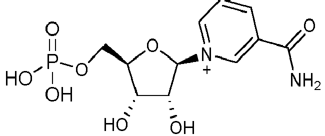 |
| I-B (alpha) | 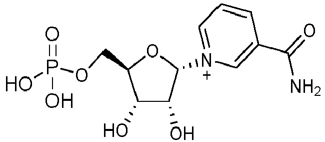 |
| I-C (beta) | 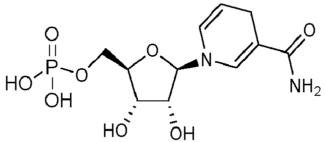 |
| I-D (alpha) | 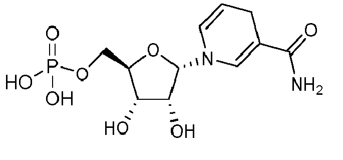 |
| I-E (beta) | 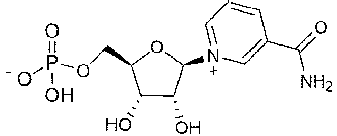 |
| I-F (alpha) | 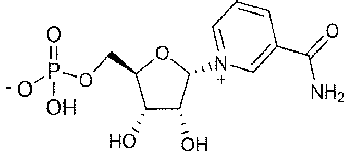 |
| I-G (beta) | 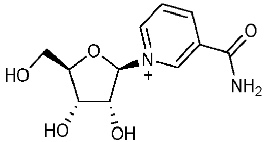 |
| I-H (alpha) | 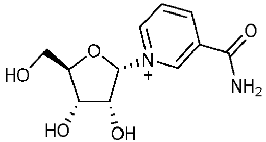 |
| I-I (beta) | 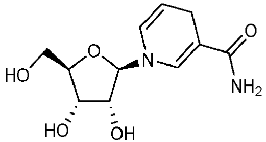 |
| I-J (alpha) | 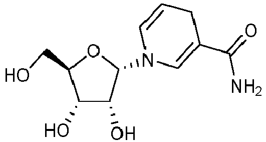 |

Advantageously, preferred compound of the invention are compounds I-A to I-F or a pharmaceutically acceptable salt or solvate thereof, more preferably IB, ID or IF.

Advantageously, the compound of formula (I) is dihydro-nicotinamide mononucleotide (NMN-H) of the following formula:

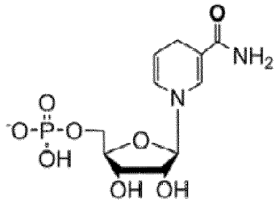

Advantageously, preferred compounds of the invention are compounds la-A to Ia-I, listed in table 2:

**[Table 2]**

| **Compound** | **Structure** |
|---|---|
| Ia-A (beta, beta) | 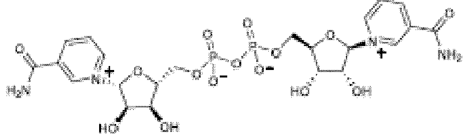 |
| Ia-B (beta, alpha) | 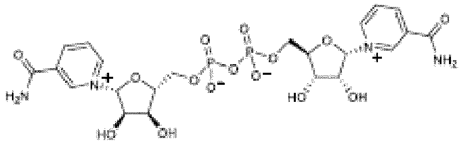 |
| la-C (alpha, alpha) | 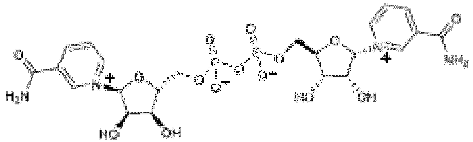 |
| Ia-D (beta, beta) | 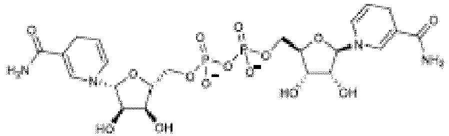 |
| Ia-E (beta, alpha) | 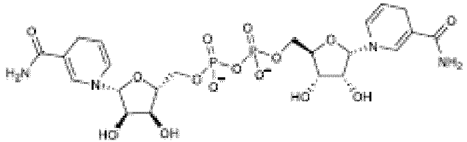 |
| Ia-F (alpha, alpha) | 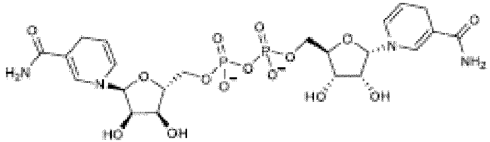 |
| Ia-G (beta, beta) | 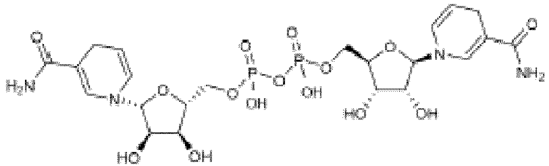 |
| Ia-H (beta, alpha) | 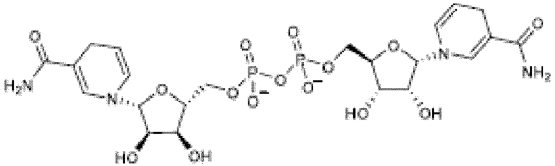 |
| Ia-I (alpha, alpha) | 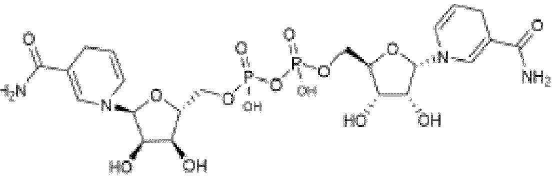 |

Advantageously, preferred compound of the invention is compound of formula la-C or la-F or Ia-I.

Advantageously, preferred compound of the invention is compound of formula la-B or la-E or la-H.

Advantageously, the improvement of sport performance is chosen amongst the improvement of maximal aerobic capacity VO2max, the improvement of the maximum heart rate HRmax, the improvement of the ventilatory threshold VT1, the improvement of the ventilatory threshold VT2, the improvement of the physical recuperation after exercise, the improvement of muscular power and their combinations.

Advantageously, the improvement of sport performance is an improvement of the VO2 max of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the VO2 max is of about 1-30%, more preferably 2-20%, even more preferably 5-15%, in a more preferred embodiment of about 10%.

Advantageously, the improvement of sport performance is an improvement of the maximum heart rate HRmax of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the maximum heart rate HRmax is by about 1-30%, preferably 2-20%, more preferably 5-15%, even more preferably of about 10%.

Advantageously, the improvement of sport performance is an improvement of the ventilatory threshold VT1 of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the ventilatory threshold VT1 is 10-70%, more preferably of about 20-50%, even more preferably of about 30-40%.

Advantageously, the improvement of sport performance is an improvement of the ventilatory threshold VT2 of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the ventilatory threshold VT2 is by about 1-30%, more preferably 2-20%, even more preferably 5-15%, in a more preferred embodiment of about 10%.

Advantageously, the improvement of sport performance is an improvement of the physical recuperation after exercise of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the physical recuperation after exercise is by about 10-70%, more preferably of about 20-50%, even more preferably of about 30-40%.

Advantageously, the improvement of sport performance is an improvement of the muscular power of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the improvement of sport performance is an improvement of the muscular power is by about 5-50%, more preferably 10-40%, even more preferably 15-25%, in a more preferred embodiment of about 20%.

Advantageously, the composition of the invention may be used in an amount between 0.1mg/day to 2000 mg/day, preferably 100 mg/day to about 1000 mg/day, more preferably about 125 to about 500 mg/day.

Advantageously, the composition of the invention may be administered orally, sublingually, parenterally, topically or by inhalation. Preferably, the composition of the invention may be administered orally or sublingually.

In an embodiment, the composition of the invention can be a pharmaceutical composition. In this embodiment, the carrier is an acceptable pharmaceutical carrier.

In an embodiment, the composition of the invention can be a dietary supplement. In this embodiment, the carrier is an acceptable food carrier.

In an embodiment, the composition of the invention can be a cosmetic composition. In this embodiment, the carrier is an acceptable cosmetic carrier.

Preferably, the composition of the invention is a dietary supplement.

Advantageously, the composition of the invention may be used in mammals, preferably humans. Advantageously, the composition of the invention may comprise at least one further ingredient. According to the invention, the at least one further ingredient can be chosen amongst amino-acids, omega-3 fatty acids, vitamins and lipids.

Advantageously, the at least one further ingredient is an amino acid is chosen amongst alanine, arginine, aspartic acid, asparagine, cysteine, glutamic acid, glutamine, glycine, histidine, isoleucine, leucine, lysine, methionine, phenylalanine, proline, serine, threonine, tryptophan, tyrosine, valine and their combinations. In a preferred embodiment, the composition of the invention may comprise at least Leucine, Isoleucine, Valine and their combination, more preferably Leucine, Isoleucine, and Valine.

Advantageously, the composition of the invention may comprise Leucine, Isoleucine, Valine, Arginine, and Glutamine.

In one embodiment, the composition of the invention comprises at least two further amino-acids chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations, wherein the combination of the at least two further amino-acids represents at least 10% by weight of the composition. In one embodiment, the composition of the invention comprises at least two further amino-acids chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations, wherein each further amino-acids represents at least 10% by weight of the composition.

Advantageously, the composition according to the invention is in the form of a tablet, a capsule, a sachet, a granule, a soft capsule, a lozenge, a lyophilisate, a suspension, a gel, a syrup, a solution, a water/oil emulsion, an oil/water emulsion, oil, cream, milk, spray, ointment, ampoule, suppository, eye drops, liquid for inhalation, dry powder inhaler, pressurised metered dose inhaler.

Preferably, the composition according to the invention is in the form of a gastro-resistant capsule or a sublingual tablet.

Advantageously, the composition according to the invention is a pharmaceutical composition. Advantageously, the composition according to the invention is a dietary supplement.

The present invention also relates to a composition comprising compound of formula I and/or compound of formula la, one of their pharmaceutically acceptable salts, at least one pharmaceutically acceptable excipient and at least one further therapeutic ingredient for its use according to the invention.

Advantageously, the at least one further therapeutic ingredient is as described herein.

The present invention also relates to a kit of parts comprising the composition of the invention and at least one further part.

Advantageously, the at least one further part may be an information leaflet.

Advantageously, the at least one further part may be at least one further ingredient as described herein. Preferably, the at least one further part comprises an information leaflet and at least one further therapeutic ingredient as described herein.

Advantageously, the kit of parts may comprise a multi-day supply of unit dosages of the composition according to the invention and instructions directing the administration of said multi-day supply over a period of multiple days.

### DEFINITIONS

The definitions and explanations below are for the terms as used throughout the entire application, including both the specification and the claims.

When describing the compounds of the invention, the terms used are to be construed in accordance with the following definitions, unless indicated otherwise.

Unless indicated otherwise, the nomenclature of substituents that are not explicitly defined herein are arrived at by naming the adjacent functionality toward the point of attachment followed by the terminal portion of the functionality. For example, the substituent "arylalkyl" refers to the group -(aryl)-(alkyl).

In the present invention, the following terms have the following meanings.

The term "alkyl" by itself or as part of another substituent refers to a hydrocarbyl radical of Formula CnH2n+1 wherein n is a number greater than or equal to 1. Generally, alkyl groups of this invention comprise from 1 to 12 carbon atoms, preferably from 1 to 8 carbon atoms, more preferably from 1 to 6 carbon atoms, still more preferably 1 to 2 carbon atoms. Alkyl groups may be linear or branched and may be substituted as indicated herein. Suitable alkyl groups include methyl, ethyl, n-propyl, i-propyl, n- butyl, i-butyl, s-butyl and t-butyl, pentyl and its isomers (e.g. n-pentyl, iso-pentyl), and hexyl and its isomers (e.g. n-hexyl, iso-hexyl). Preferred alkyl groups include methyl, ethyl, n-propyl, i-propyl, n-butyl, i-butyl, s-butyl, t-butyl, n-pentyl, n-hexyl, n-heptyl, n-octyl, n-nonyl and n-decyl. Saturated branched alkyls include, without being limited to, isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl.

Suitable alkyl groups include methyl, ethyl, n-propyl, i-propyl, n-butyl, i-butyl, s-butyl and t-butyl, pentyl and its isomers (e.g. n-pentyl, iso-pentyl), hexyl and its isomers (e.g. n-hexyl, isohexyl), heptyl and its isomers (e.g. heptyl-heptyl, iso-heptyl), octyl and its isomers (e.g. n-octyl, iso-octyl), nonyl and its isomers (e.g. n-nonyl, iso-nonyl), decyl and its isomers (e.g. n-decyl, iso-decyl), undecyl and its isomers, dodecyl and its isomers. Preferred alkyl groups are methyl, ethyl, n-propyl, i-propyl, n-butyl, i-butyl, s-butyl and t-butyl. Cx-Cy-alkyl refers to alkyl groups which comprise x to y carbon atoms, x and y being included. When the suffix "ene" ("alkylene") is used in conjunction with an alkyl group, this is intended to mean the alkyl group as defined herein having two single bonds as points of attachment to other groups. The term "alkylene" includes methylene, ethylene, methylmethylene, propylene, ethylethylene, and 1,2-dimethylethylene.

The term "alkenyl" as used herein refers to an unsaturated hydrocarbyl group, which may be linear or branched, comprising one or more carbon-carbon double bonds. Suitable alkenyl groups comprise between 2 and 12 carbon atoms, preferably between 2 and 8 carbon atoms, still more preferably between 2 and 6 carbon atoms. Examples of alkenyl groups are ethenyl, 2- propenyl, 2-butenyl, 3-butenyl, 2-pentenyl and its isomers, 2-hexenyl and its isomers, 2,4-pentadienyl and the like.

The term "alkynyl" as used herein refers to a class of monovalent unsaturated hydrocarbyl groups, wherein the unsaturation arises from the presence of one or more carbon-carbon triple bonds. Alkynyl groups typically, and preferably, have the same number of carbon atoms as described above in relation to alkenyl groups. Non limiting examples of alkynyl groups are ethynyl, 2- propynyl, 2-butynyl, 3-butynyl, 2-pentynyl and its isomers, 2-hexynyl and its isomers-and the like.

The term "aryl" as used herein refers to a polyunsaturated, aromatic hydrocarbyl group having a single ring (i.e. phenyl) or multiple aromatic rings fused together (e.g. naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non- limiting examples of aryl comprise phenyl, biphenylyl, biphenylenyl, 5- or 6-tetralinyl, naphthalen-1- or -2-yl, 4-, 5-, 6 or 7-indenyl, 1- 2-, 3-, 4- or 5- acenaphtylenyl, 3-, 4- or 5-acenaphtenyl, 1- or 2-pentalenyl, 4- or 5-indanyl, 5-, 6- , 7- or 8-tetrahydronaphthyl, 1,2,3,4-tetrahydronaphthyl, 1,4-dihydronaphthyl, 1-, 2-, 3-, 4- or 5-pyrenyl.

The term "cycloalkyl" as used herein is a cyclic alkyl group, that is to say, a monovalent, saturated, or unsaturated hydrocarbyl group having 1 or 2 cyclic structures. Cycloalkyl includes monocyclic or bicyclic hydrocarbyl groups. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, cyclohexyl, with cyclopropyl being particularly preferred.

As used herein, the term "antibody" means a polypeptide comprising a framework region from an immunoglobulin gene or fragments thereof that specifically binds and recognizes an antigen, e.g., myostatin. Use of the term antibody is meant to include whole antibodies, including single-chain whole antibodies, and antigen-binding fragments thereof. The term "antibody" includes bispecific antibodies and multispecific antibodies so long as they exhibit the desired biological activity or function.

As used herein, the term "antibody-related polypeptide" means antigen-binding antibody fragments, including single-chain antibodies, that can comprise the variable region(s) alone, or in combination, with all or part of the following polypeptide elements: hinge region, CH1, CH2, and CH3 domains of an antibody molecule. Also included in the disclosure are any combinations of variable region(s) and hinge region, CH1, CH2, and CH3 domains. Antibody- molecules of the present technology include, e.g., but are not limited to, Fab, Fab' and F(ab')2, Fd, single-chain Fvs (scFv), single-chain antibodies, disulfide-linked Fvs (sdFv) and fragments comprising either a V L or V H domain. Examples include: (i) a Fab fragment, a monovalent fragment consisting of the VL, VH, CL and CH1 domains; (ii) a F(ab')z fragment, a bivalent fragment comprising two Fab fragments linked by a disulphide bridge at the hinge region; (iii) a Fd fragment consisting of the VH and CH1 domains; (iv) a Fv fragment consisting of the VL and VH domains of a single arm of an antibody, (v) a dAb fragment (Ward, et al., Nature 341: 544-546, 1989), which consists of a V H domain; and (vi) an isolated complementarity determining region (CDR). As such "antibody fragments" can comprise a portion of a full-length antibody, generally the antigen binding or variable region thereof. Examples of antibody fragments include Fab, Fab', F(ab')2, and Fv fragments; diabodies; linear antibodies; single-chain antibody molecules; and multispecific antibodies formed from antibody fragments. Single-chain antibody molecules may comprise a polymer with a number of individual molecules, for example, dimmer, trimer or other polymers.

As used herein, the term "CDR-grafted antibody" means an antibody in which at least one CDR of an "acceptor" antibody is replaced by a CDR "graft" from a "donor" antibody possessing a desirable antigen specificity.

As used herein, the term "chimeric antibody" means an antibody in which the Fc constant region of a monoclonal antibody from one species (e.g., a mouse Fc constant region) is replaced, using recombinant DNA techniques, with an Fc constant region from an antibody of another species (e.g., a human Fc constant region). See generally Better, et al., Science 240: 1041-1043, 1988; Liu, et al., Proc Natl Acad Sci USA 84: 3439-3443, 1987; Liu, et al., J Immuno/139: 3521-3526, 1987; Sun, et al., Proc Natl Acad Sci USA 84: 214-218, 1987; Nishimura, et al., Cancer Res 47:999-1005, 1987; Wood, et al., Nature 314:446-449, 1985; and Shaw, et al., JNatl Cancer Inst 80: 1553-1559, 1988.

As used herein, the term "diabodies" refers to small antibody fragments with two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen binding sites. Diabodies are described more fully in Hollinger, et al., Proc. Natl. Acad. Sci. USA, 90: 6444-6448 (1993).

As used herein, the term "gene" means a segment of DNA that contains all the information for the regulated biosynthesis of an RNA product, including promoters, exons, introns, and other untranslated regions that control expression.

As used herein, the term "human sequence antibody" includes antibodies having variable and constant regions (if present) derived from human germline immunoglobulin sequences. The human sequence antibodies of the present technology can include amino acid residues not encoded by human germline immunoglobulin sequences (e.g., mutations introduced by random or site-specific mutagenesis in vitro or by somatic mutation in vivo). Such antibodies can be generated in non-human transgenic animals, e.g., as described in PCT Publication Nos. WO 01/14424 and WO 00/37504. However, the term "human sequence antibody," as used herein, is not intended to include antibodies in which CDR sequences derived from the germline of another mammalian species, such as a mouse, have been grafted onto human framework sequences (e.g., humanized antibodies).

As used herein, the term "humanized" forms of non-human (e.g., murine) antibodies are chimeric antibodies which contain minimal sequence derived from non-human immunoglobulin. For the most part, humanized antibodies are human immunoglobulins in which hypervariable region residues of the recipient are replaced by hypervariable region residues from a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance such as binding affinity. Generally, the humanized antibody comprises substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR are typically no more than 6 in the H chain, and in the L chain, no more than 3. The humanized antibody optionally also comprises at least a portion of an immunoglobulin constant region (Fe), typically that of a human immunoglobulin. For further details, see Jones, et al., Nature 321:522-525 (1986); Reichmann, et al., Nature 332:323-329 (1988); and Presta, Curr. Op. Struct. Bioi. 2:593- 596 (1992). As used herein, the term "hypervariable region" refers to the amino acid residues of an antibody which are responsible for antigen-binding. The hypervariable region generally comprises amino acid residues from a "complementarity determining region" or "CDR" (e.g. around about residues 24-34 (LI), 50-56 (L2) and 89-97 (L3) in the VL, and around about 31- 35B (HI), 50-65 (H2) and 95-102 (H3) in the VH (Kabat, et al., Sequences of Proteins of Immunological Interest, 5th Ed. Public Health Service, National Institutes of Health, Bethesda, MD. (1991)) and/or those residues from a "hypervariable loop" (e.g. residues 26- 32 (LI), 50-52 (L2) and 91-96 (L3) in the VL, and 26-32 (HI), 52A-55 (H2) and 96-101 (H3) in the VH (Chothia and Lesk J. Mol. Bioi. 196:901-917 (1987)).

As used herein, "gene therapy" refers to the therapeutic delivery of nucleic acids into a patient's cells as a drug to treat disease. The delivery of DNA into cells can be accomplished by multiple methods. The two major classes are recombinant viruses (sometimes called biological nanoparticles or viral vectors) and naked DNA or DNA complexes (non-viral methods).

The term "halo" or "halogen" means fluoro, chloro, bromo, or iodo. Preferred halo groups are fluoro and chloro.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoro methyl, 1,1,1-trifluoroethyl and the like. Cx-Cy-haloalkyl and Cx-Cy-alkyl are alkyl groups which comprise x to y carbon atoms. Preferred haloalkyl groups are difluoromethyl and trifluoromethyl. Where at least one carbon atom in an aryl group is replaced with a heteroatom, the resultant ring is referred to herein as a heteroaryl ring.

The term "heteroalkyl" means an alkyl group as defined above in which one or more carbon atoms are replaced by a heteroatom selected from oxygen, nitrogen and sulfur atoms. In heteroalkyl groups, the heteroatoms are linked along the alkyl chain only to carbon atoms, i.e. each heteroatom is separated from any other heteroatom by at least one carbon atom. However, the nitrogen and sulphur heteroatoms may optionally be oxidised and the nitrogen heteroatoms may optionally be quaternised. A heteroalkyl is bonded to another group or molecule only through a carbon atom, i.e. the bonding atom is not selected from the heteroatoms included in the heteroalkyl group.

The term "heteroaryl" as used herein by itself or as part of another group refers but is not limited to 5 to 12 carbon-atom aromatic rings or ring systems containing 1 to 2 rings which are fused together or linked covalently, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2, 1 -b] [1 ,3] thiazolyl, thieno [3 ,2-b] furanyl, thieno [3 ,2-b] thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2- benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3- benzoxadiazolyl, 1 ,2,3-benzothiadiazolyl, 2, 1 ,3-benzothiadiazolyl, thienopyridinyl, purinyl, imidazo[1,2-a]pyridinyl, 6-oxo-pyridazin-1(6H)-yl, 2- oxopyridin-1(2H)-yl, 6-oxo-pyridazin-1(6H)-yl, 2-oxopyridin-1(2H)-yl, 1,3- benzodioxolyl, quinolinyl, isoquinolinyl, cinnolinyl, quinazolinyl, quinoxalinyl.

Where at least one carbon atom in a cycloalkyl group is replaced with a heteroatom, the resultant ring is referred to herein as "heterocycloalkyl" or "heterocyclyl".

The terms "heterocyclyl", "heterocycloalkyl" or "heterocyclo" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. Any of the carbon atoms of the heterocyclic group may be substituted by oxo (for example piperidone, pyrrolidinone). The heterocyclic group may be attached at any heteroatom or carbon atom of the ring or ring system, where valence allows. The rings of multi- ring heterocycles may be fused, bridged and/or joined through one or more spiro atoms. Non limiting exemplary heterocyclic groups include oxetanyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, 3H-indolyl, indolinyl, isoindolinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2- oxopiperidinyl, 2-oxopyrrolodinyl, indolinyl, tetrahydropyranyl, tetrahydrofuranyl, tetrahydroquinolinyl, tetrahydroisoquinolin- 1-yl, tetrahydroisoquinolin-2-yl, tetrahydroisoquinolin-3-yl, tetrahydroisoquinolin-4-yl, thiomorpholin-4-yl, thiomorpholin-4-ylsulf oxide, thiomorpholin-4-ylsulfone, 1,3- dioxolanyl, 1,4-oxathianyl, IH-pyrrolizinyl, tetrahydro-l,l-dioxothiophenyl, N- formylpiperazinyl, and morpholin-4-yl.

The term "non-proteinogenic amino acid" as used herein refers to an amino acid not naturally encoded or found in the genetic code of living organism. Non limiting examples of Non-proteinogenic amino acid are ornithine, citrulline, argininosuccinate, homoserine, homocysteine, cysteine-sulfinic acid, 2-aminomuconic acid, δ-aminolevulinic acid, β-alanine, cystathionine, γ-aminobutyrate, DOPA, 5-hydroxytryptophan, D-serine, ibotenic acid, α-aminobutyrate, 2-aminoisobutyrate, D-leucine, D-valine, D-alanine or D-glutamate .

The term "proteinogenic amino acid" as used herein refers to an amino acid that is incorporated into proteins during translation of messenger RNA by ribosomes in living organisms, i.e. Alanine (ALA), Arginine (ARG), Asparagine (ASN), Aspartate (ASP), Cysteine (CYS), Glutamate (glutamic acid) (GLU), Glutamine (GLN), Glycine (GLY), Histidine (HIS), Isoleucine (ILE), Leucine (LEU), Lysine (LYS), Methionine (MET), Phenylalanine (PHE), Proline (PRO), Pyrrolysine (PYL), Selenocysteine (SEL), Serine (SER), Threonine (THR), Tryptophan (TRP), Tyrosine (TYR) or Valine (VAL).

The term "prodrug" as used herein means the pharmacologically acceptable derivatives of compounds of formula (I) such as esters whose in vivo biotransformation product is the active drug. Prodrugs are characterized by increased bio-availability and are readily metabolized into the active compounds in vivo. Suitable prodrugs for the purpose of the invention include carboxylic esters, in particular alkyl esters, aryl esters, acyloxyalkyl esters, and dioxolene carboxylic esters; ascorbic acid esters.

The term "substituent" or "substituted" means that a hydrogen radical on a compound or group is replaced by any desired group which is substantially stable under the reaction conditions in an unprotected form or when protected by a protecting group. Examples of preferred substituents include, without being limited to, halogen (chloro, iodo, bromo, or fluoro); alkyl; alkenyl; alkynyl, as described above; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl), monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO2CH3; CONH2; OCH2CONH2; NH2; SO2NH2; OCHF2; CF3; OCF3; and such moieties may also be optionally substituted by a fused-ring structure or bridge, for example -OCH2O-. These substituents may optionally be further substituted with a substituent selected from such groups. In certain embodiments, the term "substituent" or the adjective "substituted" refers to a substituent selected from the group consisting of an alkyl, an alkenyl, an alkynyl, an cycloalkyl, an cycloalkenyl, a heterocycloalkyl, an aryl, a heteroaryl, an arylalkyl, a heteroarylalkyl, a haloalkyl, -C(O)NR11R12, -NR13C(O)R14, a halo, -OR13, cyano, nitro, a haloalkoxy, -C(O)R13, -NR11R12, -SR13, -C(O)OR13, -OC(O)R13, -NR13C(O)NR11R12, -OC(O)NR11R12, -NR13C(O)OR14, -S(O)rR13, -NR13S(O)rR14, -OS(O)rR14, S(O)rNR11R12, -O, -S, and -N-R13, wherein r is 1 or 2; R11 and R12, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl; or R11 and R12 taken together with the nitrogen to which they are attached is optionally substituted heterocycloalkyl or optionally substituted heteroaryl; and R13 and R14 for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl. In certain embodiments, the term "substituent" or the adjective "substituted" refers to a solubilizing group. The term "active ingredient" or "therapeutic ingredient" refers to a molecule or a substance whose administration to a subject slows down or stops the progression, aggravation, or deterioration of one or more symptoms of a disease, or condition; alleviates the symptoms of a disease or condition; cures a disease or condition. According to one embodiment, the therapeutic ingredient is a small molecule, either natural or synthetic. According to another the therapeutic ingredient is a biological molecule such as for example an oligonucleotide, a siRNA, a miRNA, a DNA fragment, an aptamer, an antibody and the like. By "pharmaceutically acceptable" is meant that the ingredients of a pharmaceutical composition are compatible with each other and not deleterious to the patient thereof.

The term "pharmaceutically acceptable excipient" or "pharmaceutical vehicle" refers to an inert medium or carrier used as a solvent or diluent in which the pharmaceutically active agent is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as the FDA or EMA. For the purposes of the invention, "pharmaceutically acceptable excipient" includes all pharmaceutically acceptable excipients as well as all pharmaceutically acceptable carriers, diluents, and/or adjuvants.

The term "pharmaceutically acceptable salts" include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.

Hemisalts of acids and bases may also be formed, for example, hemisulphate and hemicalcium salts. Pharmaceutically acceptable salts of compounds of Formula (I) may be prepared by one or more of these methods:
(i) by reacting the compound of Formula (I) with the desired acid;
(ii) by reacting the compound of Formula (I) with the desired base;
(iii) by removing an acid- or base-labile protecting group from a suitable precursor of the compound of Formula (I) or by ring-opening a suitable cyclic precursor, e.g., a lactone or lactam, using the desired acid; and/or
(iv) by converting one salt of the compound of Formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

Although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of Formula I above.

The term "solvate" is used herein to describe a molecular complex comprising a compound of the invention and contains stoichiometric or sub-stoichiometric amounts of one or more pharmaceutically acceptable solvent molecule, such as ethanol. The term 'hydrate' refers to when said solvent is water. The term "administration", or a variant thereof (e.g., "administering"), means providing the active agent or active ingredient, alone or as part of a pharmaceutically acceptable composition, to the patient in whom/which the condition, symptom, or disease is to be treated or prevented.

The term "human" refers to a subject of both genders and at any stage of development (i.e., neonate, infant, juvenile, adolescent, adult).

The term "patient" refers to a warm-blooded animal, more preferably a human, who/which is awaiting the receipt of, or is receiving medical care or is/will be the object of a medical procedure.

The terms "treat", "treating" and "treatment", as used herein, are meant to include alleviating, attenuating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient that is sufficient to achieve the desired therapeutic or prophylactic effect in the patient to which/whom it is administered.

The bonds of an asymmetric carbon can be represented here using a solid triangle ( 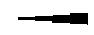 ), a dashed triangle ( 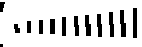 ) or a zigzag line ( 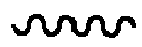 ).

### DETAILED DESCRIPTION OF THE INVENTION

The present invention thus relates to a compound of formula (I): 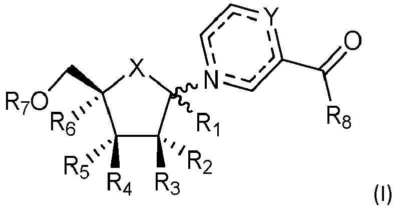 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 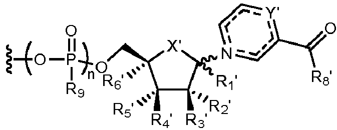 wherein n is an integer chosen amongst 1 or 3; wherein:
R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
   R11 is selected from C1-C10 alkyl, C3-C10 cycloalkyl, C5-C18 aryl, C1-C10 alkylaryl, substituted C5-C12 aryl, C1-C10 heteroalkyl, C3₋C10 heterocycloalkyle, C1-C10 haloalkyl, heteroaryl, -(CH2)nC(O)(C1-Cl5)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of Rlla is independently selected from H and (C1-C6) alkyl and Rllb is independently selected from OH, C1-C6 alkoxy, NH2, NH(C1-C6 alkyl) or N(C1-C6 alkyl)2 ;
   R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
   R13 and R14 are independently selected from H, C1-C8 alkyl and C1-C8 alkyl aryl;
   Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
   R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
   R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl ;
   Y is selected from CH, CH2, C(CH3)2 and CCH3;
   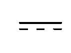 represents a single or double bond according to Y; and
   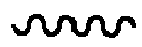 represents the alpha or beta anomer depending on the position of R1,
   or a compound of formula (1a) 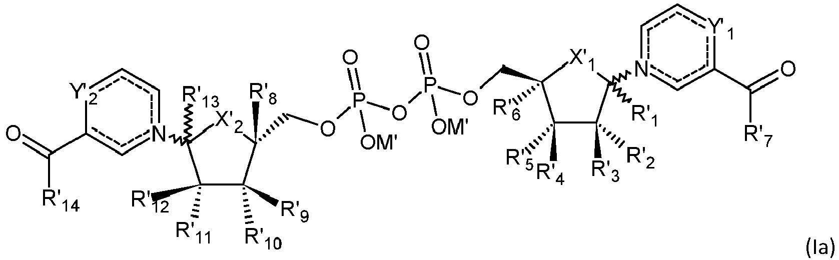
   or pharmaceutically acceptable salts, hydrates, crystals, stereoisomers and/or solvates thereof, wherein:
      X'1 and X'2 are independently selected from O, CH2, S, Se, CHF, CF2 and C=CH2;
      R'1 and R'13 are independently selected from H, azido, cyano, C1-C8 alkyl, C1-C8 thio-alkyl, C1-C8 heteroalkyl and OR; wherein R is selected from H and C1-C8 alkyl;
      R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
      R'6 and R'8 are independently selected from H, azido, cyano, C1-C8 alkyl and OR; wherein R is selected from H and C1-C8 alkyl;
      R'7 and R'14 are independently selected from H, OR, NHR, NRR', NH-NHR, SH, CN, N3 and halogen;
      wherein R and R' are each independently selected from H, C1-C8 alkyl, C1-C8 alkyl aryl;
      Y'1 and Y'2 are independently selected from CH, CH2, C(CH3)2 or CCH3;
      M' is selected from H or a suitable counter-ion;
      -̅-̅-̅ represents a single or a double bound depending on Y'1 and Y'2; and
      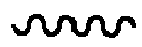 represents the alpha or beta anomer depending on the position of R'1 and R'13,
      and their combinations and at least one carrier, for use in the improvement of sport performance.

Compounds of formula I and formula Ia are derivatives of nicotinamide mononucleotide. More specifically, compounds of formula I comprises NMN and its derivatives. Compounds of formula Ia are derivatives of nicotinamide mononucleotide (NMN).

Advantageously, X is selected from O, CH2 and S.

Advantageously, R1 is selected from hydrogen or OH. In one embodiment, R1 is hydrogen. In one embodiment, R1 is OH.

Advantageously, R2, R3, R4 and R5 are independently selected from hydrogen, halogen, hydroxyl, C1-C12 alkyl and OR; wherein R is as described herein above. In a preferred embodiment, R2, R3, R4 and R5 are independently selected from hydrogen, hydroxyl and OR; wherein R is as described herein above. In a more preferred embodiment R2, R3, R4 and R5 are independently selected from hydrogen or OH.

Advantageously, R2 and R3 are identical. In one embodiment, R2 and R3 are identical and represent OH. In one embodiment, R2 and R3 are identical and represent hydrogen.

Advantageously, R2 and R3 are different. In a preferred embodiment, R2 is hydrogen and R3 is OH. In a more preferred embodiment, R2 is OH and R3 is hydrogen.

Advantageously, R4 and R5 are identical. In one embodiment, R4 and R5 are identical and represent OH. In one embodiment, R4 and R5 are identical and represent hydrogen.

Advantageously, R2 and R3 are different. In a preferred embodiment, R4 is OH and R5 is hydrogen. In a more preferred embodiment, R4 is hydrogen and R5 is OH.

Advantageously, R3 and R4 are different. In one embodiment, R3 is OH and R4 is hydrogen. In one embodiment, R3 is hydrogen and R4 is OH.

Advantageously, R3 and R4 are identical. In a preferred embodiment, R3 and R4 are identical and represent OH. In a more preferred embodiment, R3 and R4 are identical and represent hydrogen.

Advantageously, R2 and R5 are different. In one embodiment, R2 is hydrogen and R5 is OH. In one embodiment, R2 is OH and R5 is hydrogen.

According to one embodiment, R2 and R5 are identical. In a preferred embodiment, R2 and R5 are identical and represent hydrogen. In a more preferred embodiment, R2 and R5 are identical and represent OH.

According to one embodiment, R6 is selected from hydrogen or OH. In one embodiment, R6 is OH. In a preferred embodiment, R6 is hydrogen.

Advantageously, R7 is selected from P(O)R9R10 or P(S)R9R10; wherein R9 and R10 are as described herein above. In a preferred embodiment, R7 is P(O)R9R10; wherein R9 and R10 are as described herein above. In a preferred embodiment, R7 is P(O)(OH)2.

Advantageously, R8 is selected from H, OR, NHR13 or NR13R14; wherein R13 and R14 are as described herein above. In a preferred embodiment, R8 is NHR13; wherein R13 and R14 are as described herein above.

Advantageously, Y is a CH or CH2. In one embodiment, Y is a CH. In one embodiment, Y is a CH2. According to one preferred embodiment, compounds of formula (I) are those wherein X is an oxygen.

According to a preferred embodiment, the invention relates to compounds of general Formula (II): 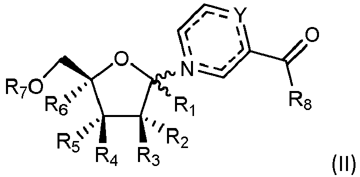 or a pharmaceutically acceptable salt or solvate thereof; wherein R1, R2, R3, R4, R5, R6, R7, R8, Y, 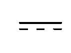 and 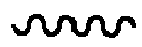 are as described herein above for compounds of formula (I).

Advantageously, preferred compounds of formula (I) are those wherein R1 is hydrogen.

Advantageously, preferred compounds of formula (I) are those wherein R2 is OH and R3 is hydrogen.

Advantageously, preferred compounds of formula (I) are those wherein R4 is hydrogen and R5 is OH.

Advantageously, preferred compounds of formula (I) are those wherein R3 and R4 are identical and represent hydrogen.

Advantageously, preferred compounds of formula (I) are those wherein R2 and R5 are identical and represent OH.

According to one embodiment, preferred compounds of formula (I) are those wherein R6 is hydrogen.

Advantageously, preferred compounds of formula (I) are those wherein R8 is NH2.

Advantageously, preferred compounds of formula (I) are those wherein Y is CH.

Advantageously, preferred compounds of formula (I) are those wherein Y is CH2.

Advantageously, preferred compounds of formula (I) are those wherein R7 is P(O)(OH)2.

Advantageously, the compound according to the invention is selected from compounds I-A to I-J from Table 1 below ci-dessous or a pharmaceutically acceptable salt or solvate thereof:

**[Table 1]**

| **Composes (anomères)** | **Structure** |
|---|---|
| I-A (beta) | 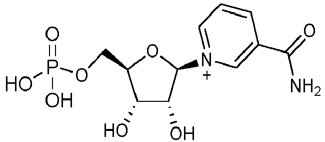 |
| I-B (alpha) | 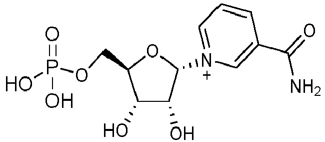 |
| I-C (beta) | 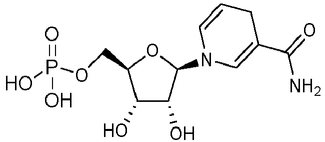 |
| I-D (alpha) | 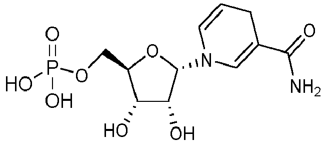 |
| I-E (beta) | 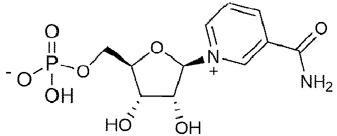 |
| I-F (alpha) | 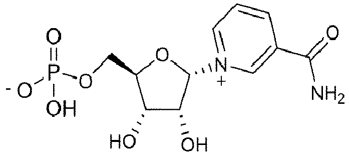 |
| I-G (beta) | 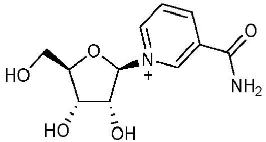 |
| I-H (alpha) | 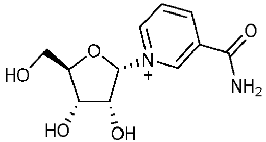 |
| I-I (beta) | 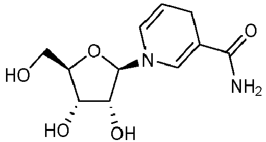 |
| I-J (alpha) | 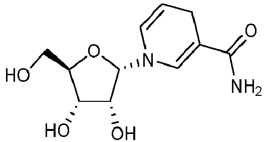 |

Advantageously, preferred compound of the invention are compounds I-A to I-F or a pharmaceutically acceptable salt or solvate thereof.

Advantageously, the pharmaceutically acceptable derivative is NMN-H of the following formula:

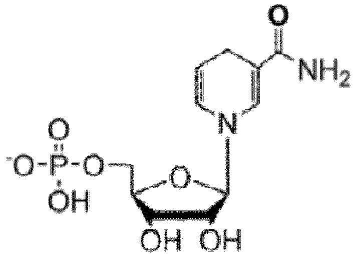

Advantageously, preferred compounds of general Formula Ia are those wherein X'1 and X'2 are independently selected from O, CH2, S.

Advantageously, R'7 and R'14 are independently selected from H, OR, NHR and NRR' wherein R and R' are independently selected from H, C1-C8 alkyl, C1-C8 alkyl aryl. According to one embodiment, R'7 and R'14 are NHR wherein R is selected from H, C1-C8 alkyl, C1-C8 alkyl aryl.

Advantageously, R'2, R'3, R'4, R'5, R'9, R'10, R'11, R'12 are independently selected from H, halogen, hydroxyl, C1-C12 alkyl and OR. According to a preferred embodiment, R'2, R'3, R'4, R'5, R'9, R'10, R'11, R'12 are independently selected from H, hydroxyl and OR, wherein R is as described herein above.

Advantageously, preferred compounds of general Formula Ia are those wherein, R'2, R'3, R'4, R'5, R'9, R'10, R'11, R'12 are independently selected from H and OH.

Advantageously, R'2 and R'3 are identical. According to one embodiment, R'2 and R'3 are identical and represent each a OH. According to one embodiment, R'2 and R'3 are identical and represent each hydrogen.

According to a preferred embodiment, R'2 and R'3 are different. According to a preferred embodiment, R'2 is hydrogen and R'3 is a OH. According to a more preferred embodiment, R'2 is a OH and R'3 is hydrogen.

Advantageously, R'4 and R'5 are identical. According to one embodiment, R'4 and R'5 are identical and represent each a OH. According to one embodiment, R'4 and R'5 are identical and represent each hydrogen.

According to a preferred embodiment, R'4 and R'5 are different. According to a preferred embodiment, R'4 is a OH and R'5 is hydrogen. According to a more preferred embodiment, R'4 is hydrogen and R'5 is a OH.

Advantageously, R'3 and R'4 are identical. According to one embodiment, R'3 and R'4 are identical and represent each a OH. According to one embodiment, R'3 and R'4 are identical and represent each hydrogen.

According to a preferred embodiment, R'3 and R'4 are different. According to a preferred embodiment, R'3 is a OH and R'4 is hydrogen. According to a more preferred embodiment, R'3 is hydrogen and R'4 is a OH

Advantageously, R'2 and R'5 are different. According to one embodiment, R'2 is hydrogen and R'5 is a OH. According to one embodiment, R'2 is a OH and R'5 is hydrogen.

According to a preferred embodiment, R'2 and R'5 are identical. According to a preferred embodiment, R'2 and R'5 are identical and represent each hydrogen. According to a more preferred embodiment, R'2 and R'5 are identical and represent each a OH.

Advantageously, R'9 and R'10 are identical. According to one embodiment, R'9 and R'10 are identical and represent each a OH. According to one embodiment, R'9 and R'10 are identical and represent each hydrogen.

According to a preferred embodiment, R'9 and R'10 are different. According to a preferred embodiment, R'9 is hydrogen and R'10 is a OH. According to a more preferred embodiment, R'9 is a OH and R'10 is hydrogen.

Advantageously, R'11 and R'12 are identical. According to one embodiment, R'11 and R'12 are identical and represent each a OH. According to one embodiment, R'11 and R'12 are identical and represent each hydrogen.

According to a preferred embodiment, R'11 and R'12 are different. According to a preferred embodiment, R'11 is a OH and R'12 is hydrogen. According to a more preferred embodiment, R'11 is hydrogen and R'12 is a OH.

Advantageously, R'10 and R'11 are different. According to one embodiment, R'10 is hydrogen and R'11 is a OH. According to one embodiment, R'10 is a OH and R'11 is hydrogen.

According to a preferred embodiment, R'10 and R'11 are identical. According to a preferred embodiment, R'10 and R'11 are identical and represent each a OH. According to a more preferred embodiment, R'10 and R'11 are identical and represent each hydrogen.

Advantageously, R'9 and R'12 are different. According to one embodiment, R'9 is hydrogen and R'12 is a OH. According to one embodiment, R'9 is a OH and R'12 is hydrogen.

According to a preferred embodiment, R'9 and R'12 are identical. According to a preferred embodiment, R'9 and R'12 are identical and represent each hydrogen. According to a more preferred embodiment, R'9 and R'12 are identical and represent each a OH.

Advantageously, Y'1 is CH. According to one embodiment, Y'1 is CH2.

Advantageously, Y'2 is CH. According to one embodiment, Y'2 is CH2.

Advantageously, X'1 and X'2 are different and are selected from the group as described above. According to one embodiment, X'1 and X'2 are identical and are selected from the group as described above.

Advantageously, preferred compounds of general Formula Ia are those wherein X'1 and X'2 each independently represents an Oxygen.

Advantageously, preferred compounds of general Formula Ia are those wherein X'1 and X'2 are identical and represent each an Oxygen.

Advantageously, R'7 and R'14 are different and are selected from the group as described above. According to one embodiment, R'7 and R'14 are identical and are selected from the group as described above.

Advantageously, preferred compounds of general Formula Ia are those wherein R'7 and R'14 each independently represents a NH2.

Advantageously, preferred compounds of general Formula Ia are those wherein R'7 and R'14 are identical and represent each a NH2.

Advantageously, R'1 and R'13 are different and are selected from the group as described above. According to one embodiment, R'1 and R'13 are identical and are selected from the group as described above.

Advantageously, preferred compounds of general Formula Ia are those wherein R'1 and R'13 each independently represents a hydrogen.

Advantageously, preferred compounds of general Formula Ia are those wherein R'1 and R'13 are identical and represent each a hydrogen.

Advantageously, R'6 and R'8 are different and are selected from the group as described above. According to one embodiment, R'6 and R'8 are identical and are selected from the group as described above.

Advantageously, preferred compounds of general Formula Ia are those wherein R'6 and R'8 each independently represents a hydrogen.

Advantageously, preferred compounds of general Formula Ia are those wherein R'6 and R'8 are identical and represent each a hydrogen.

Advantageously, R'3, R'4, R'10 and R'11 are different and are selected from the group as described above. According one embodiment, R'3, R'4, R'10 and R'11 are identical and are selected from the group as described above.

Advantageously, preferred compounds of general Formula Ia are those wherein R'3, R'4, R'10 and R'11 each independently represents a hydrogen.

Advantageously, preferred compounds of general Formula Ia are those wherein R'3, R'4, R'10, R'11 are identical and represent each a H.

Advantageously, R'2, R'5, R'9 and R'12 are different and are selected from the group as described above. According one embodiment, R'2, R'5, R'9 and R'12 are identical and are selected from the group as described above.

Advantageously, preferred compounds of general Formula Ia are those wherein R'2, R'5, R'9 and R'12 each independently represents a OH.

Advantageously, preferred compounds of general Formula Ia are those wherein R'2, R'5, R'9, R'12 are identical and represent each a OH.

Advantageously, Y'1 and Y'2 are different. According to a preferred embodiment, Y'1 and Y'2 are identical. Advantageously, preferred compounds of general Formula Ia are those wherein Y'1 and Y'2 each independently represents a CH.

Advantageously, preferred compounds of general Formula Ia are those wherein Y'1 and Y'2 are identical and represent each a CH.

Advantageously, preferred compounds of general Formula Ia are those wherein Y'1 and Y'2 each independently represents a CH2.

Advantageously, preferred compounds of general Formula Ia are those wherein Y'1 and Y'2 are identical and represent each a CH2.

Advantageously, preferred compounds of the invention are compounds Ia-A to Ia-I, listed in table 2:

**[Table 2]**

| **Compound** | **Structure** |
|---|---|
| Ia-A (beta, beta) | 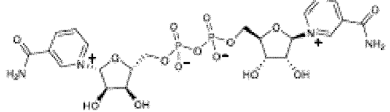 |
| Ia-B (beta, alpha) | 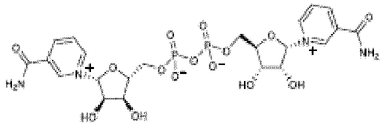 |
| Ia-C (alpha, alpha) | 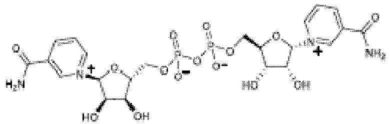 |
| Ia-D (beta, beta) | 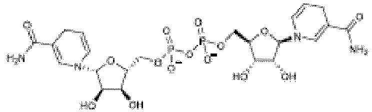 |
| Ia-E (beta, alpha) | 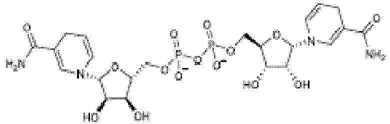 |
| Ia-F (alpha, alpha) | 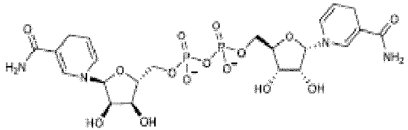 |
| Ia-G (beta, beta) | 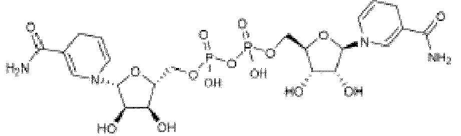 |
| Ia-H (beta, alpha) | 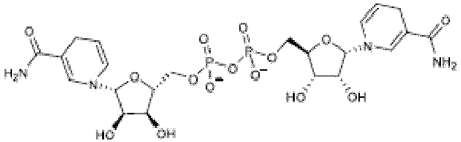 |
| Ia-I (alpha, alpha) | 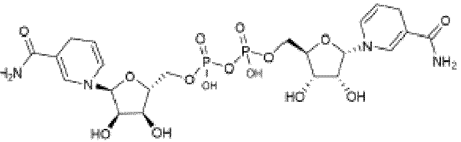 |

Advantageously, preferred compound of the invention is compound of formula la-C or la-F or Ia-I.

Advantageously, preferred compound of the invention is compound of formula la-B or la-E or la-H. All references to compounds of Formula (I) or (1a) include references to salts, solvates, multi component complexes and liquid crystals thereof. All references to compounds of Formula (I) or (1a) include references to polymorphs and crystal habits thereof.

### Sport performance

Compositions according to the invention can be used for the improvement of sport performance. In the description herein, the expression "sport performance", "athletic performance", or "physical performance" can be used as synonyms. The physical capacity is the ability to continue a physical activity despite increasing physical or psychological stress. It refers to the time elapsed before reaching a given level of fatigue during physical exercise under predefined conditions (e.g. at a given load, intensity or speed). Increasing physical capacity is considered a beneficial physical effect by EFSA, particularly in the context of resuming a sporting activity but also to contribute to the continuation of daily physical activity. Advantageously, the improvement of sport performance is chosen amongst the improvement of maximal aerobic capacity VO2max, the improvement of the maximum heart rate HRmax, the improvement of the ventilatory threshold VT1, the improvement of the ventilatory threshold VT2, the improvement of the physical recuperation after exercise, the improvement of muscular power and their combinations.

The VO2max is the maximum amount of oxygen that the body can use per unit of time. It is the quantity of oxygen that the lungs can breathe in and transfer to the blood, and that the muscles will have to capture. It is expressed in terms of the following 2 different units: in L or mL of oxygen per minute (L/min) or per kilogram of body weight (mL/min/kg). The first unit (L/min) is called "absolute" while the second (mL/min/kg) is called "relative" or "standardized" and is used to compare two athletes of different sizes. VO2 max can be measured notably by running on a treadmill in a laboratory with a gas analysis machine that can measure the subject's oxygen consumption in real time. The VO2max is a key factor for the improvement of endurance. VO2max can be improved, for instance, by running at a long time at high speed. The inventors have found out that the compositions of the invention were suitable to improve the VO2max of the subject of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the compositions of the invention can improve the VO2max by about 1-30%, more preferably 2-20%, even more preferably 5-15%, in a more preferred embodiment of about 10%. Therefore, the compositions of the invention are useful for helping an athlete, whether professional or occasional, to improve his oxygenation capacity.

Maximum heart rate HRmax is the amount of beats a heart makes in a minute under maximum stress. Max HR is used as a benchmark for maximum output the athlete's body can produce. Knowing that number enables the athlete and his coach to structure the training process around specific training intensities or 'training zones'. There are several methods to calculate the HRmax. The most common is HRmax = 220-age of the patient. It can also be calculated in a supervised laboratory test wherein the athlete runs on a treadmill (or cycles/kayaks/etc. on an ergometer) with an ever-increasing speed/power until complete exhaustion. The inventors have found out that the compositions of the invention could improve the maximal heart rate of the subject of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the compositions of the invention can improve improve the maximal heart rate of the subject by about 1-30%, preferably 2-20%, more preferably 5-15%, even more preferably of about 10%.

Ventilatory threshold 1 (VT1) is a marker of intensity that can be observed in a person's breathing at a point where lactate begins to accumulate in the blood. The ventilatory threshold 1 can also be designated as the aerobic ventilatory threshold (AVE), the ventilatory threshold, the aerobic threshold or the aerobic gas exchange threshold. As the intensity of the exercise begins to increase, VT1 can be identified at the point where the breathing rate begins to increase. A person who is at VT1 can no longer talk comfortably but can still string together a few words-while exercising. Ventilatory threshold 2 (VT2) is higher marker of VT1 and corresponds to the stage wherein the lactate has quickly accumulated in the blood and the person needs to breathe heavily. The ventilatory threshold 2 can also be designated as the respiratory compensation threshold (RCP), the anaerobic ventilatory threshold or the anaerobic aerobic gas exchange threshold. At this rapid rate of breathing, the exerciser can no longer speak. The exercise duration will necessarily decrease due to the intensity level. VT2 can also be called the anaerobic threshold or lactate threshold. The composition of the invention can improve the ventilatory threshold VT1 of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the composition of the invention can improve the ventilatory threshold VT1 of the subject of about 10-70%, more preferably of about 20-50%, even more preferably of about 30-40%. The composition of the invention can improve the ventilatory threshold VT2 of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the composition of the invention can improve the ventilatory threshold VT2 of the subject by about 1-30%, more preferably 2-20%, even more preferably 5-15%, in a more preferred embodiment of about 10%.

The physical recuperation after exercise also reflects the physical capacity of an athlete: the faster the athlete recovers, the better is his physical capacity or sport performance is. The physical recuperation after exercise can be measured by measuring the performance in Watt, recorded during a Wingate test. The Wingate typically involves 30 seconds of maximal exercise on either an arm-crank or legcycle ergometer. The testing device is a mechanically braked cycle ergometer. Following a five-minute warm-up, which includes three sprints at varying resistances, the athlete may get off the bike during a three-minute recovery or stay on the bike and spin lightly. The athlete then begins to pedal as fast as possible without any or minimal resistance. Within three seconds, a fixed resistance is applied to the flywheel and the athlete continues to pedal "all out" for the duration of the test (e.g. 30 seconds). The compositions of the invention can improve the physical recuperation after exercise of at least 1%, at least 2%, at least 3%, at least 4%, at least 5%, at least, 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the composition of the invention can improve the physical recuperation after exercise of the subject by about 10-70%, more preferably of about 20-50%, even more preferably of about 30-40%.

Muscular power refers to a great force production over a short period of time, such as in fast leg kicks and explosive jumping. Muscular power is different from muscular strength, which refers to the ability to exert maximal force in one single contraction, or to muscular endurance which is when less force is sustained over a longer period of time. The composition of the invention can improve the muscular power of at least 1%, at least 2%, at least 3%, at least 4%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. Preferably, the composition of the invention can improve the muscular power of the subject by about 5-50%, more preferably 10-40%, even more preferably 15-25%, in a more preferred embodiment of about 20%. In an embodiment, the compositions according to the invention are to be used in mammals, preferably humans. In a preferred embodiment, the human is an athlete. In an embodiment, the compounds of formula (I) or formula (1a) and compositions according to the invention are to be used in male humans. In an embodiment, the compounds of formula (I) and/or compounds of formula (1a) and compositions according to the invention are to be used in patients aged 18 or less than 18.

### Combinations with at least one further ingredient

Compositions of the invention can be used in combinations with one or more further ingredients. The further ingredient or ingredients are preferably selected amongst amino-acids, omega 3 fatty acids, vitamins or minerals, antioxidants and their combinations. The antioxidants can be chosen amongst resveratrol, urolithine A, urolithine B, fisetine, folinic acid, alpha lipoic acid co-enzyme Q10, pyrroloquinoleine quinone and their combinations.

The at least one amino acid can be chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations. Preferably, the composition of the invention may comprise at least one amino-acid chosen amongst Leucine, Isoleucine, Valine and their combination and/or at least one of Arginine, Glutamine, L-Carnithine, Citrulline, Creatine and their combination. More preferably, the composition of the invention comprises Leucine, Isoleucine, and Valine. Leucine, Isoleucine and Valine are branched-chain amino-acids and are beneficial to prevent catabolism during exercise and increase the recovery after exercise. In a most preferred combination, the composition of the invention comprises Leucine, Isoleucine, Valine, Arginine, and Glutamine.

In one embodiment, the composition of the invention comprises at least two further amino-acids chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations, wherein the combination of the at least two further amino-acids represents at least 10% by weight of the composition. In one embodiment, the composition of the invention comprises at least two further amino-acids chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations, wherein each further amino-acids represents at least 10% by weight of the composition.

The composition can comprise Leucine in an amount of 200-2000 mg/day, preferably between 500-1500 mg/day, more preferably 1000 mg/day.

The composition can comprise Isoleucine in an amount of 50-1500 mg/day, preferably between 250-1000 mg/day, more preferably 500 mg/day.

The composition can comprise Valine in an amount of 50-1500 mg/day, preferably between 250-1000 mg/day, more preferably 500 mg/day.

The composition can comprise Arginine in an amount of 50-1500 mg/day, preferably between 250-1000 mg/day, more preferably 500 mg/day.

The composition can comprise Glutamine in an amount of 50-1500 mg/day, preferably between 250-1000 mg/day, more preferably 750 mg/day.

Vitamins can be selected amongst vitamin B1, vitamin B2, vitamin B3, vitamin B5, vitamin B6, vitamin B2, vitamin B8, vitamin B9, vitamin B12, vitamin C, vitamin E, vitamin K3 and their combinations. Preferably, the vitamin can be vitamin B1, vitamin B2, vitamin B6, vitamin C and their combination.

Minerals can be selected amongst calcium, phosphate, magnesium, iron, zinc, chromium, boron, vanadium, selenium and their combinations. Preferably, the mineral is chosen amongst chromium, iron, magnesium and their combinations.

The omega 3 fatty acids can be alpha-linolenic acid (ALA), eicosapentaenoic acid (EPA), docosahexaenoic acid (DHA) and their combinations.

Compounds and compositions according to the invention can be administered with the further ingredient simultaneously, separately, or sequentially. By "simultaneously" is meant that two agents are administered concurrently. By "separately" is meant that the gap between the administration of the first agent and that of the second is substantial. By "sequentially" is meant that the two agents are administered one after the other within a time frame such that they are both available to act therapeutically within the same time frame. The optimum time interval between administering the two agents will vary depending on the precise nature of the method for delivering the compounds or compositions of the invention.

### Compositions

The present invention also relates to a composition comprising compound of formula I and/or compound of formula Ia as described hereinabove and at least one acceptable carrier for the improvement of sport performance.

In one embodiment, the composition according to the invention is a pharmaceutical composition. In another embodiment, the composition according to the invention is a dietary supplement. Preferably, the composition according to the invention is a pharmaceutical composition.

In one embodiment, the pharmaceutical composition according to the invention may be used in combination with at least one further therapeutic ingredient as described herein. In another embodiment, the pharmaceutical composition according to the invention comprises as least one further therapeutic ingredient as described herein.

The compositions according to the invention may be formulated with carriers, excipients and diluents which are suitable in themselves for these formulations, such as lactose, dextrose, saccharose, sorbitol, mannitol, starches, acacia gum, calcium phosphate, alginates, tragacanth gum, gelatine, calcium silicate, microcrystalline cellulose, polyvinylpyrrolidone, polyethylene glycol, cellulose, water (sterile), methyl cellulose, methyl and propyl hydroxybenzoates, talc, magnesium stearate, edible oils, vegetable and mineral oils or suitable mixtures thereof. The formulations may possibly contain other substances that are commonly used in pharmaceutical formulations, such as lubricants, wetting agents, emulsifying and suspending agents, dispersing agents, disintegrants, fillers, preservatives, sweeteners, flavourings, flow regulators, mould release agents, etc. The compositions can also be formulated to allow a rapid, prolonged or delayed release of the active compound(s) they contain.

The compositions according to the invention are preferably in the form of unit doses and may be packaged in an appropriate manner, for example in a box, blister pack, bottle, sachet, ampoule or in any other suitable single-dose or multiple-dose carrier or container (which may be properly labelled); optionally with one or more leaflet(s) containing product information and/or instructions for use.

Compositions of the invention can be prepared by any method known by the skilled in the art.

### Methods of administration

The compounds of formula (I) and formula (1a) as well as compositions of the invention as described herein, may be administered by oral, parenteral (e.g., intramuscular, intraperitoneal, intravenous, intracerebroventricular, intracisternal injection or infusion, subcutaneous injection, or implant), by inhalation spray, nasal, rectal, sublingual, or topical routes of administration and may be formulated, alone or together, in suitable dosage unit formulations containing conventional non-toxic acceptable carriers, adjuvants and vehicles appropriate for each route of administration. In addition to the treatment of warm-blooded animals, such as mice, rats, horses, cattle, sheep, dogs, cats, monkeys, etc., the compounds of the invention are effective for use in humans. Preferably, the composition of the invention may be used in mammals, more preferably humans.

In an embodiment, the composition of the invention can be a pharmaceutical composition. In this embodiment, the carrier is an acceptable pharmaceutical carrier. In another embodiment, the composition of the invention can be a dietary supplement. In this embodiment, the carrier is an acceptable food carrier. In another embodiment, the composition of the invention can be a cosmetic composition. In this embodiment, the carrier is an acceptable cosmetic carrier. Preferably, the composition of the invention is a dietary supplement.

The compositions of the invention may conveniently be presented in dosage unit form and may be prepared by any of the methods well known in the art of pharmacy or food industry. All methods include the step of bringing the active ingredient into association with the carrier which constitutes one or more accessory ingredients. In general, the compositions are prepared by uniformly and intimately bringing the active ingredient into association with a liquid carrier or a finely divided solid carrier or both, and then, if necessary, shaping the product into the desired formulation. In pharmaceutical compositions, the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

Preferably, the composition of the invention may be administered orally or sublingually. The compositions in a form suitable for oral use can be administered, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material, such as glyceryl monostearate or glyceryl distearate may be employed. They may also be coated by the techniques described in the U.S. Patents 4,256,108; 4,166,452; and 4,265,874 to form osmotic therapeutic tablets for control release. Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy- propylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol , such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin. Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an anti-oxidant, such as ascorbic acid. Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Syrups and elixirs may be formulated with sweetening agents, for example glycerol, propylene glycol, sorbitol or sucrose. Such formulations may also contain a demulcent, a preservative and flavouring and colouring agents.

The compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. For topical use, creams, ointments, jellies, solutions or suspensions, etc., containing the compounds of the present invention are employed. (For purposes of this application, topical application shall include mouthwashes and gargles.)

An appropriate dosage level is generally about 0.1 to 2000 mg per day which can be administered in single or multiple doses. Preferably, the dosage level is about 100 to about 1000 mg/day, more preferably about 125 to about 500 mg/day. Within this range the dosage may be 50 mg/day; 125 mg/day; 250 mg/day; 500 mg/day. For oral administration, the compositions are preferably provided in the form of tablets containing 0.1 to 1000 milligrams of the active ingredient, particularly 1.0, 5.0, 10.0, 15.0, 20.0, 25.0, 50.0, 75.0, 100.0, 125.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient. For example, the dosage may comprise from 50mg/day to 2000mg/day, preferably from 125mg/day to 1000mg/day.

"Gastro-resistant" means a galenic form that does not dissolve in the stomach. Such dosage forms are delayed release, i.e. they have a coating or coating composition that is resistant to the acid pH of the stomach (pH<2) to dissolve in the intestine. The gastroresistant character is determined by following the test established by the European Pharmacopoeia. Briefly, the gastroresistant character of a capsule is measured in 0.1 M hydrochloric acid at 37°C as a disintegrating medium in a disintegrating apparatus. This medium mimics the physicochemical conditions of the stomach. The capsules are incubated in this medium for 1 hour. The capsule must show no signs of disintegration or cracks that could lead to a loss of contents. The capsule is then incubated for 1 hour in a phosphate buffer solution of pH 6.8 at 37°C, this solution mimics the conditions of the intestinal environment in accordance with the recommendations of the European Pharmacopoeia. The capsule must be completely disintegrated within one hour.

"Sublingual tablet" means a galenic form that is placed under the tongue so that the active principle is absorbed by the sublingual mucosa, and in particular by the ranine veins and arteries.

The galenic form of the composition according to the invention may also be immediate release: such a galenic form allows rapid absorption of NMN, its salts or derivative, and thus a reduced time of action. Immediate release galenic forms include dispersible, orodispersible, effervescent tablets and oral lyophilisates.

The galenic form of the composition according to the invention may also be delayed release. Dissolution and absorption of the compounds of formula (I) and/or formula (1a) of the invention takes place in the intestine, which limits gastric irritation or degradation of the fragile active ingredients at acidic pH. These are mainly gastroresistant forms, i.e. the tablets or granules are covered with a polymeric film, insoluble in an acidic medium but permeable to water in an alkaline medium or of the lipidic type degraded by intestinal lipases.

The galenic form of the composition according to the invention may also be a prolonged and sequential release form. The forms with sequential release (release at precise time intervals) and prolonged release (continuous release of the active principle until exhaustion) promote the spreading of the release of the active principle over time in order to maintain an effective plasma concentration for a longer period of time in the patient's body. Such galenic forms make it possible to obtain relief of the patient's pain for a longer period of time and to space out the medication intake.

The mode of administration and the galenic form are determined by the person in the profession according to the anatomical location of the pain to be treated and the patient. Reference is made to the latest edition of Remington's Pharmaceutical Sciences.

Compositions according to the invention may be administered between 1 to 4 times per day, preferably once, twice or three times per day. Once or twice a day has been found suitable. Preferably, the compounds and compositions of the invention are to be taken before breakfast.

The duration of the treatment depends on the subject. It can be from one day to one year or even longer, preferably from one week to three months, more preferably from two weeks to six weeks. It will be understood, however, that the specific dose level and frequency of dosage and duration for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### Kit of parts

The present invention also relates to a kit of parts comprising at least a composition of the invention as described hereinabove, and at least one further part.

In one embodiment, the at least one further part may be an information leaflet which may comprise information to the user and/or instructions for use. In one embodiment, the at least one further part may be at least one further ingredient as set out above. Preferably, the at least one further part comprises an information leaflet and at least one further therapeutic ingredient as described herein. The kit of parts of the invention may comprise a multi-day supply of unit dosages of the composition according to the invention and instructions directing the administration of said multi-day supply over a period of multiple days. The kit of parts may be used in the improvement of sport performance as described herein.

### Synthesis of compounds of the invention

According to another aspect, the invention relates to a method for the preparation of the compound of Formula (I) as described above. Compounds of formula (I) can also be prepared according to the methods disclosed in international patent application WO 2017/079195 A1, US patent US 10,611,790 B2 and US patent US 10,618,927 B1.

In particular, the compounds of Formula (I) disclosed herein may be prepared as described below from substrates A-E. It shall be understood by a person skilled in the art that these schemes are in no way limiting and that variations may be made without departing from the spirit and scope of this invention.

The method can involve in a first step the mono-phosphorylation of a compound of formula (A), in the presence of phosphoryl chloride and a trialkyl phosphate, to yield the phosphorodichloridate of formula (B), 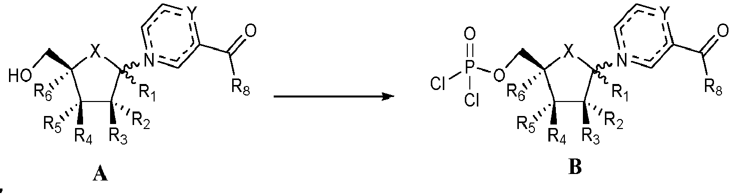 wherein X, R1, R2, R3, R4, R5, R6, R7, R8, Y, 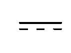 and 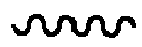 are as described herein above for compounds of formula (I).

In a second step, the phosphorodichloridate of formula (B) is hydrolysed to yield the phosphate of formula (C), 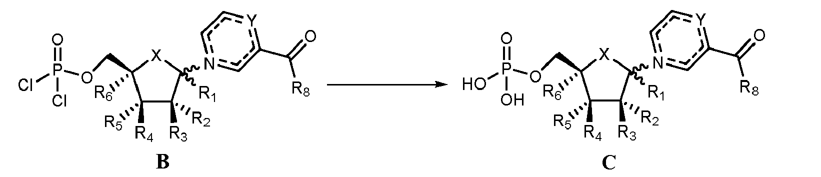 wherein X, R1, R2, R3, R4, R5, R6, R7, R8, Y, 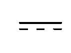 and 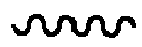 are as described herein above for compounds of formula (I).

The compound of formula (A) can be synthesized using various methods known to the person skilled in the art. According to one embodiment, the compound of formula (A) is synthesized by reacting the pentose of formula (D) with a nitrogen derivative of formula (E), wherein R, R2, R3, R4, R5, R6, R7, Y are as described above for compounds of formula (I), leading to the compound of formula (A-1) which is then selectively deprotected to give the compound of formula (A), 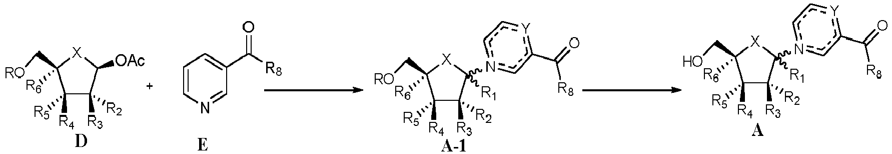 wherein X, R1, R2, R3, R4, R5, R6, R8, Y, 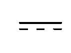 and 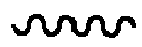 are as described herein above for compounds of formula (I).

R can be an appropriate protective group known to the skilled person in the art. In one embodiment, the protecting group is selected from triarylmethyls and/or silyls. Non-limiting examples of triarylmethyl include trityl, monomethoxytrityl, 4,4'-dimethoxytrityl and 4,4',4"-trimethoxytrityl. Non-limiting examples of silyl groups include trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

Any hydroxyl group can be attached to the pentose is protected by an appropriate protective group known to the person skilled in the art.

The choice and exchange of protective groups is the responsibility of the person skilled in the art. Protective groups can also be removed by methods well known to the skilled person, for example, with an acid (e.g. mineral or organic acid), base or fluoride source. According to a preferred embodiment, the nitrogen derivative of formula (E) is coupled to the pentose of formula (D) by a reaction in the presence of a Lewis acid leading to the compound of formula (A-1). Non-limiting examples of Lewis acids include TMSOTf, BF3.OEt2, TiCl4 and FeCl3.

The method of the present invention can further comprise a step of reducing the compound of formula (A) by various methods well known to the skilled person in the art, leading to the compound of formula (A') wherein is CH2 and R1, R2, R3, R4, R5, R6, R8, Y, 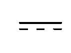 and 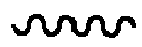 are as defined above for compounds of formula (I).

According to a specific embodiment, the present invention relates to a method for the preparation of the compounds of formula I-A to I-D.

In a first step, the nicotinamide of formula E is coupled to the ribose tetraacetate of formula D by a coupling reaction in the presence of a Lewis acid, resulting in the compound of formula A-1:

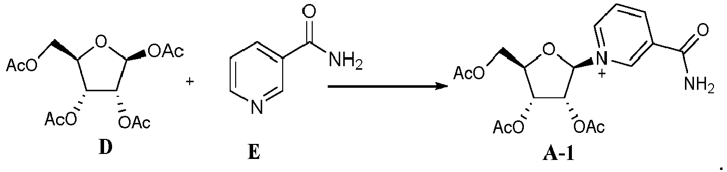

In a second step, an ammoniacal treatment of the compound of formula A-1 is carried out, leading to the compound of formula A-2:

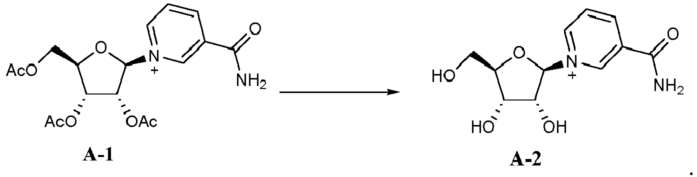

In a third step, the mono-phosphorylation of the compound of formula A-2, in the presence of phosphoryl chloride and a trialkyl phosphate, leads to the phosphorodichloridate of formula A-3:

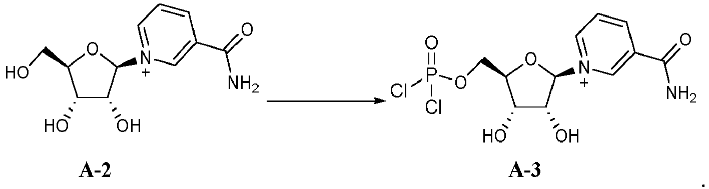

In a fourth step, the phosphorodichloridate of formula A-3 is hydrolysed to yield the compound of formula I-A:

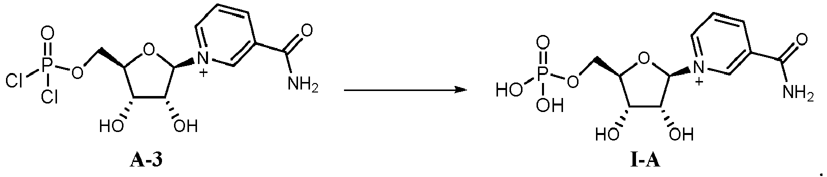

According to one embodiment, a step of reducing the compound of formula A-2 is carried out, leading to the compound of formula I-E of formula:

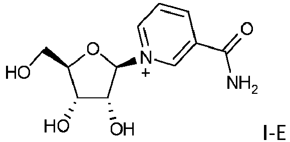

The compound of formula I-E is then monophosphorylated as described in step 4 and hydrolyzed to the compound of formula I-C.

In another aspect, the invention relates to a method for preparing compounds of formula Ia as described above.

In particular, compounds of formula Ia disclosed herein can be prepared as described below from substrates Xa-Xllla. It will be understood by one ordinary skilled in the art that these schemes are in no way limiting and that variations of detail can be made without departing from the spirit and scope of the present invention.

According to one embodiment, the invention relates to a method for preparing the compound of formula I described herein above.

The method first involves the mono-phosphorylation of a compound of formula Xa, in the presence of phosphoryl chloride in a trialkyl phosphate, to give the phophorodichloridate compound Xla, 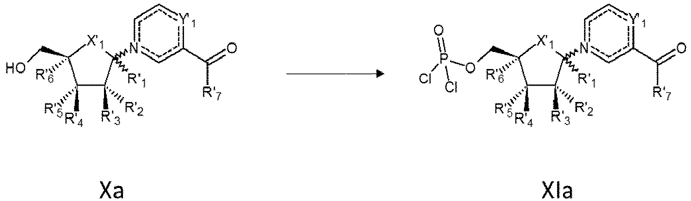 wherein X'1, R'1, R'2, R'3, R'4, R'5, R'6, R'7, Y'1, 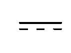 and 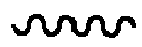 are as described herein for formula Ia.

In a second step the hydrolysis of the phophorodichloridate XIa obtained in the first step give the phosphate compound of formula Xlla, 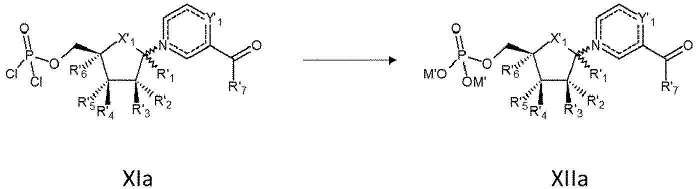 wherein X'1, R'1, R'2, R'3, R'4, R'5, R'6, R'7, Y'1, M', 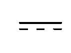 and 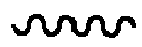 are as described herein for formula Ia. The phosphate compound of formula Xlla obtained in the second step is then reacted, with a phophorodichloridate compound of formula XIIIa obtained as described in the first step, 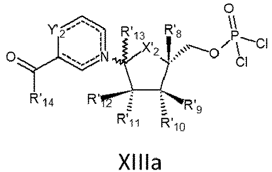 wherein X'2, R'8, R'9, R'10, R'11, R'12, R'13, R'14, Y'2, 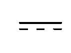 and 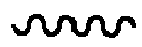 are as are as described herein for formula la, to give the compound of formula Ia as described herein.

According to one embodiment, the method of the invention further comprises a step of reducing the compound of formula la, using various methods known to those skilled in the art, to give the compound of formula I'a, wherein Y'1 and Y'2 are identical and represent each CH2 and wherein X'1, X'2, R'1, R'2, R'3, R'4, R'5, R'6, R'7, R'8, R'9, R'10, R'11, R'12, R'13, R'14, Y'1, Y'2, M', 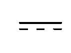 and 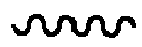 are as described herein for formula Ia.

According to another embodiment, the compound of formula Xa is synthesized using various methods known to those skilled in the art. According to one embodiment, the compound of formula Xa is synthesized in two steps by first reacting the pentose of formula XIVa with the nitrogenous derivatives of formula XVa, wherein R'1, R'1, R'2, R'3, R'4, R'5, R'6, R'7, Y'1 and R are as described herein for formula Ia, to give the compound of formula Xa-1, then selectively deprotected to give the compound of formula Xa. 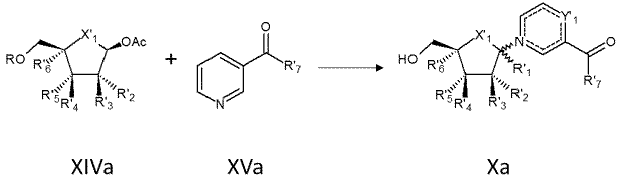 wherein X'1, R, R'1, R'2, R'3, R'4, R'5, R'6, R'7, Y'1, 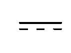 and 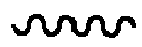 are as described herein for formula Ia.

According to one embodiment, R is an appropriate protecting group known to those skilled in the art. Examples of appropriate protecting group includes triarylmethyl and/or silyl groups. Non limiting examples of triarylmethyl includes trityl, monomethoxytrityl, 4,4'-dimethoxytrityl and 4,4',4"-trimethoxytrityl. Non limiting examples of silyl groups includes trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

According to one embodiment, any hydroxy group attached to the pentose ring is protected with an appropriate protecting group known to those skilled in the art.

The selection and exchange of the protecting groups is within the skill to those skilled in the art. Any protecting groups can also be removed by methods known in the art, for example, with an acid (e.g., a mineral or an organic acid), a base or a fluoride source.

According to a preferred embodiment, the nitrogenous derivatives of formula XVa is added to the pentose XIVa via a coupling reaction in the presence of a Lewis acid to give the compound of formula Xa-1. Non limiting examples of suitable Lewis acid includes TMSOTf, BF3.OEt2, TiCl4 and FeCl3.

According to a specific embodiment, the invention relates to a method for preparing the compound of formula Villa, 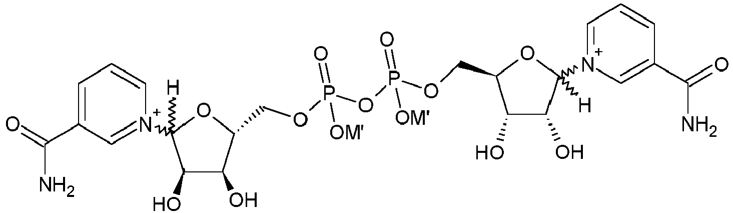 or pharmaceutically acceptable salts and/or solvates thereof.

In a first step, the nicotinamide of formula XVa, is added to the ribose tetraacetate XIVa, via a coupling reaction in the presence of a Lewis acid, to give the compound of formula Xa-1:

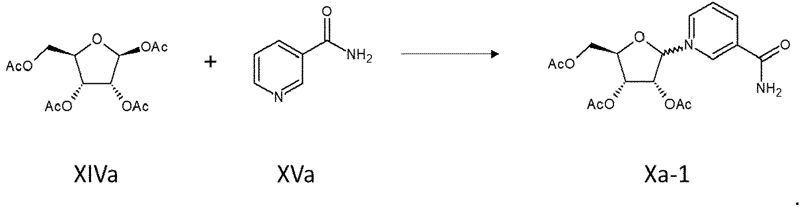

In a second step, an ammoniacal treatment of the compound of formula Xa-1 give the compound of formula Xa:

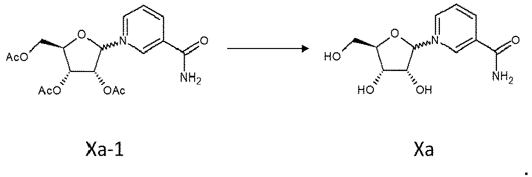

In a third step, the mono-phosphorylation of a compound of formula Xa, in the presence of phosphoryl chloride in a trialkyl phosphate, give the phophorodichloridate compound Xla:

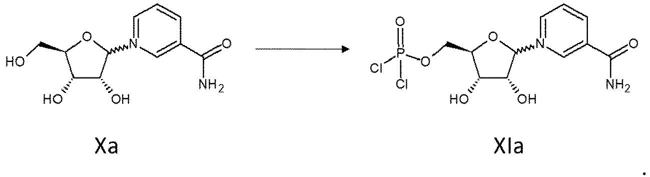

In a fourth step, the phophorodichloridate compound XIa obtained in the third step is partially hydrolyzed to give the phosphate compound of formula Xlla:

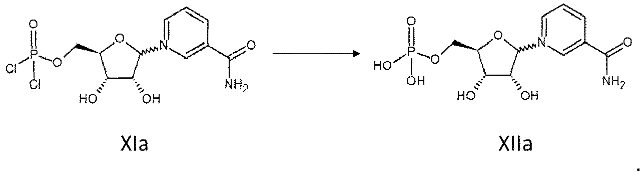

In a fifth step, the phosphate compound of formula XIIa obtained in the fourth step is then reacted, with the phophorodichloridate compound of formula XIa obtained as described in the third step, to give the compound of formula Villa.

According to another specific embodiment, the invention relates to a method for preparing the compound of formula IXa, 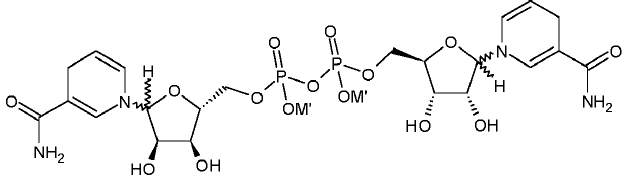 or pharmaceutically acceptable salts and/or solvates thereof.

According to one embodiment, the compound of formula IXa is obtained from the compound of formula Villa, previously synthesized as described above.

In this embodiment, the compound of formula IXa is obtained by reducing the compound of formula Villa, using a suitable reducing agent known to those skilled in the art, to give the compound of formula IXa.

### EXAM PLES

In the following description, the examples are provided to illustrate the present invention and are in no way intended to limit its scope.

### Example 1 - Dietary supplement of the invention

An embodiment of a dietary supplement according to the invention is disclosed herein. The composition of the invention can be prepared by mixing all the elements together according to any conventional method known to the skilled in the art. The dietary supplement of the invention is formulated as a powder. In this embodiment, the dietary supplement is packaged in a unit dose, such as a stick, comprising 5g of composition as detailed below:

**Table 3**

| Ingredient | Amount per 5g-serving |
|---|---|
| L-Leucine (Leu) | 1000 mg |
| L-Isoleucine (Ile) | 500 mg |
| L-Valine (Val) | 500 mg |
| L-Glutamine (Gln) | 750 mg |
| L-Arginine (Arg) | 500 mg |
| Beta-NMN (compound I-A) | 125 mg |
| Natural flavor | 662,5 mg |
| Citric Acid | 700 mg |
| Calcium silicate | 50 mg |
| Malic acid | 150 mg |
| Stevia | 50 mg |
| Natural color | 12,5 mg |

The dietary supplement according to the invention can be administered once daily.

### Example 2 - Improvement of the sport performance in a 36-year-old professional tennis player.

A male professional tennis player, aged 36, has been administered 500 mg beta NMN (compound I-A) for 7 weeks. The subject was asked to carry out his training as usual without altering it in any way with the introduction of a new cardio or muscle strength routine.

The evolution of his sport performance and body composition has been assessed every week by the measuring of the following parameters:
A) Mass composition:
   - body mass (kg)
   - muscular mass (kg)
   - fat mass (kg)
B) Aerobic performance
   - Maximal aerobic power (MAP in Watt)
   - Maximal heart rate (HRmax in bpm)
   - Heart recuperation (HRmax after 1 min exercise in bpm)
   - Ventilatory threshold 1 (VT1)
   - Ventilatory threshold 2 (VT2)
   - Maximal oxygen consumption (VO2max)

The evolution of the subject's mass composition and physical performance during the test is summarized below:

**Table 4**

| Parameter | Week 1 | Week 2 | Week 3 | Week 4 | Week 5 | Week 6 | Week 7 | Evolution |
|---|---|---|---|---|---|---|---|---|
| Body mass | 103.2 | 103.9 | 102.8 | 103 | 103.2 | 102.2 | 103.1 | -0.1% |
| Muscular mass | 46.9 | 46.2 | 46.8 | 45.8 | 45.4 | 45.9 | 45.8 | -2.3% |
| Fat mass | 20.4 | 22.1 | 20.2 | 22.1 | 22.89 | 21.5 | 22.3 | 1.9 % |
| VO2 max | 40 | 44.5 | 39 | 40 | 38.7 | 44.4 | 44.5 | 11.3 % |
| HR Max | 170 | 174 | 171 | 177 | 167 | 187 | 186 | 9.4% |
| HR max after 1 min | 17 | 23 | 25 | 27 | 27 | 18 | 22 | 29.4% |
| MAP | 275 | 300 | 275 | 300 | 275 | 325 | 325 | 18.2% |
| VT1 | 150 | 150 | 175 | 175 | 150 | 200 | 200 | 33.3% |
| VT2 | 225 | 250 | 250 | 250 | 225 | 250 | 250 | 11.1% |

As it can be seen from the results summarized in Table 4, the dietary supplement of the invention did not significantly alter the subject's body composition. However, his sport performance has been greatly improved. Notably, maximal aerobic power, reflecting his muscular power, improved by almost 20% in 7 weeks. The VT1 also improved by 33.3% and his VO2max and VT2 improved by more than 10% which reflect the improvement of his physical endurance. Indeed, even with a high intensity training, the usual improvement of VO2 max is comprised between 5-15%. The composition of the invention thus improves significantly the sport performance of the subject.

### Example 3 - Randomised double-blind clinical trial of Nicotinamide mononucleotide on muscle recovery and physical capacity in healthy volunteers with moderate physical activity

The purpose of the study is to evaluate the efficacy of the daily administration of a composition of the invention, in the form of a tablet, comprising either 250 mg beta NMN (total dosage for 2 tablets) or 500 mg beta NMN (total dosage for 2 tablets). The trial comprises three groups of patients:
- Placebo: maltodextrin, Magnesium stearate
- Group NMN 1: 250 mg/day (for 2 tablets)
- Group NMN 2: 500 mg/day (for 2 tablets)

More specifically, a dietary supplement according to the invention containing 250 mg/day of beta-NMN and 500 mg/day of beta-NMN, to be taken in 2 tablets every morning. Each tablet comprises isomalt and magnesium stearate. The placebo product is identical in appearance but contains only maltodextrin instead of NMN. Each subject will receive either NMN 250 mg/day or NMN 500 mg/day or placebo according to randomisation throughout the study.

Inclusion criteria: The following subjects can be included in the trial:
Healthy male volunteers:
- Aged 20 to 49 (range limits included);
- Subjects with a BMI between 20 and 28 (range limits included);
- Subjects with a body mass between 70 kg and 100 kg (range limits included);
- Having given written informed consent to their participation;
- Moderately physically active according to the GPAQ (Global Physical Activity Questionnaire);
- Having a sporting practice involving endurance or split runs (including team or individual sports) or cycling; and
- Accepting not to change one's physical activity habits throughout the study.

### Non-inclusion criteria:

Subjects may not be included in the trial:
- Presenting a psychological state that does not allow them to give a free and informed consent to their participation in the study;
- Regularly taking or have taken in the last month any medication or dietary supplement likely to increase endurance, recovery or physical capacity. In particular, the following drugs are strictly forbidden: beta-2 agonists such as salbutamol (Ventoline^{®}), terbutaline (Bricanyl^{®}), fenoterol (Berotec^{®}), salmeterol (Serevent^{®}) and formoterol (Foradil^{®}) as well as corticosteroids;
- Presenting a recent (less than 3 months) lower limb muscle injury;
- Presenting a depressive syndrome;
- Presenting, in the opinion of the investigating physician, any symptomatology, pathology or cardiovascular risk factors incompatible with the conduct of the study or the performance of an exercise test or which could influence the results of the study; and in particular: abnormal resting and/or exercise ECG at the inclusion visit according to the investigator; presence of abnormal lipid (TC, TG, HDL, LDL), blood glucose, and hemodynamic parameters; Abnormal laboratory findings (Creatinine, Uric acid, Troponin, ALT/ASAT, LDH, Creatine Kinase, C-reactive Protein, Transferrin, Bilirubin, Gamma GT, Cholesterol (EAL), Blood glucose, lonogram, PAL);
- Subject may not be compliant with the constraints imposed by the protocol;
- Having an allergy or contraindication to the components of the products studied;
- Subject involved in another clinical trial or in the exclusion period of a previous clinical trial;
- Subject unable to understand, or read fluently; Subject not affiliated to a health insurance fund; Vulnerable persons or persons deprived of their liberty by a judicial or administrative decision ;
- Subjects using illegal psychotropic substances or having a drinking more than 2 glasses of alcohol/wine per day.

The main criterion of the study is muscle recovery assessed by measuring performance (in Watts recorded during the 30 s of pedaling) in a Wingate ergocycle test performed after an endurance test and recovery time at D42. The average power developed after 30s and Peak Power Output (PPO) (= force (kg) x distance (m) ÷ time (s)) every 5 seconds is noted.

Secondary endpoints include:
- Muscle recovery assessed by measuring performance (in Watts recorded during the 30 s of pedalling) during a Wingate test on an ergocycle carried out after an endurance test and a recovery time at D25;
- Physical capacity assessed in an endurance test by the maximum duration (time limit) of sustained exercise in minutes on a treadmill at a power corresponding to 85% of the maximum aerobic speed (MAS) determined in an exercise test;
- Cardiorespiratory recovery after exercise as assessed by changes in blood pressure and heart rate parameters at rest and at several minute intervals after the endurance test;
- Perception of the difficulty of the effort assessed by the Borg scale during the endurance test;
- Change in lactatemia before and after the endurance test measured with a finger from a 5 µL blood sample;
- Perception of the intensity of post-exercise muscle pain (cramps) using a 7-point numerical scale 24, 48 and 72 hours after the endurance and muscle recovery tests;
- Body composition determined by impedancemetry (fat mass, muscle mass, visceral fat, water mass);
- Metabolomic analyses by taking blood and urine samples.

Example 4 - Synthesis of compound of formula I wherein Synthesis of compounds of formula I wherein R7 is 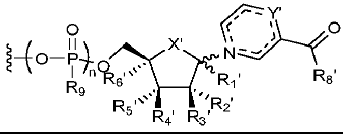 and n is 1:
As disclosed herein, a compound of formula I wherein R7 is 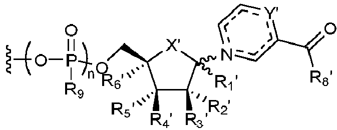 and n is 1, the synthesis can involve in a first step the mono-phosphorylation of alpha-nicotinamide riboside, in the presence of phosphoryl chloride and a trialkyl phosphate, to yield the phosphorodichloridate:

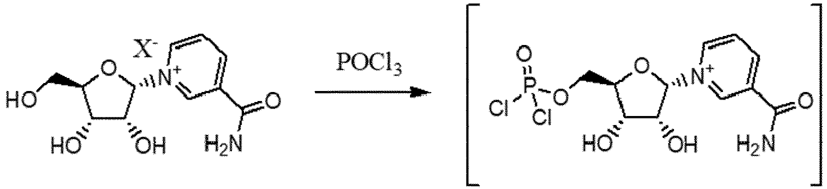

Compound I-B (alpha NMN) is then added as follows:

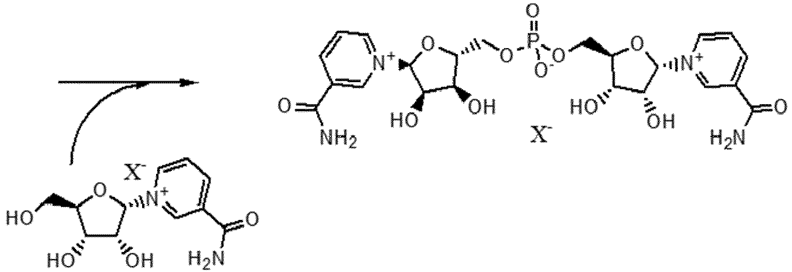

Alternatively, the compound according to the invention can be prepared by the activation of compound I-B by the addition of carbonyldiimidazole (CDI): 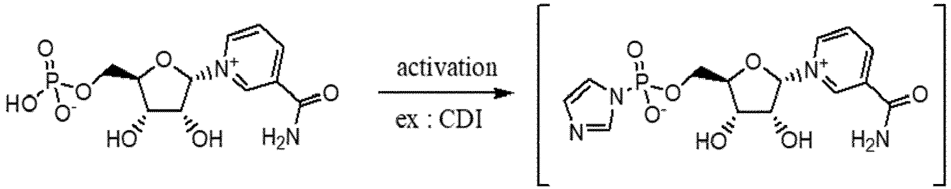 to which alpha -nicotinamide riboside is added as follows:

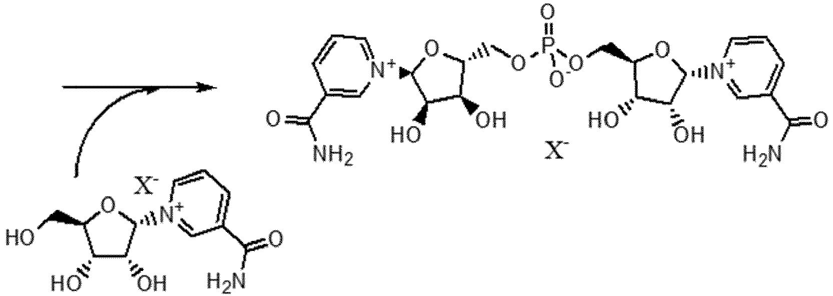

Example 5 - Synthesis of compound of formula I wherein Synthesis of compounds of formula I wherein R7 is 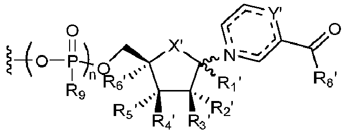 and n is 3:
A compound of formula I comprising three phosphate groups can be prepared as follows. Compound I-B can be activated by the addition of carbonyldiimidazole (CDI):

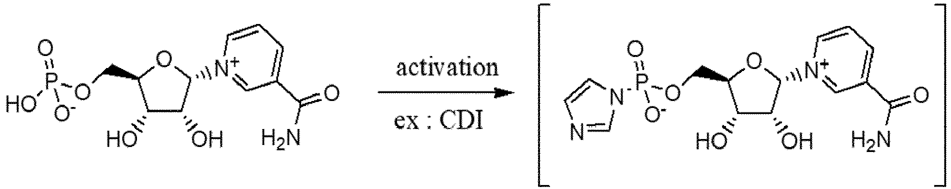

To which a tertbutyleamine -phosphate is added:

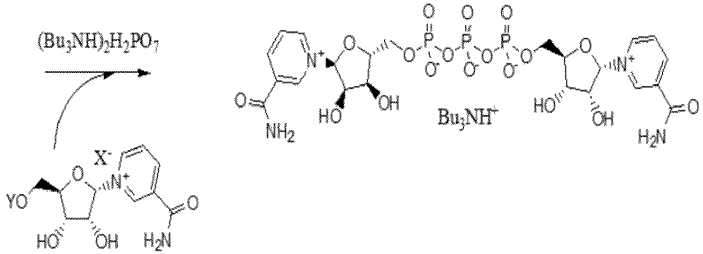

## Claims

1. Composition comprising a compound of formula (I): 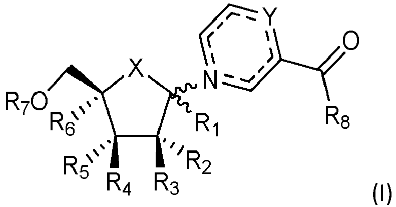 or pharmaceutically acceptable salts, hydrates, crystals, stereoisomers and/or solvates thereof, wherein:
- X is selected from O, CH2, S, Se, CHF, CF2 et C=CH2;
- R1 is selected from H, azido, cyano, C1-C8 alkyl, C1-C8 thio-alkyl, C1-C8 heteroalkyl and OR; wherein R is selected from H and C1 C8 alkyl;
- R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
- R6 is selected from H, azido, cyano, C1-C8 alkyl, C1-C8 thio-alkyl, C1-C8 heteroalkyl and OR; wherein R is selected from H and C1 C8 alkyl;
- R7 is selected from P(O)R9R10, P(S)R9R10 and 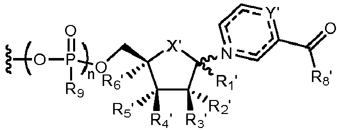 wherein n is an integer chosen amongst 1, 2 or 3; wherein:
- R9 and R10 are independently selected from 0-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12;
wherein:
- R11 is selected from C1-C10 alkyl, C3-C10 cycloalkyl, C5-C18 aryl, C1-C10 alkylaryl, substituted C5-C12 aryl, C1-C10 heteroalkyl, C3-C10 heterocycloalkyle, C1-C10 haloalkyl, heteroaryl, -(CH2)nC(O)(C1-C15)alkyl, -(CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of Rlla is independently selected from H and (C1-C6) alkyl and Rllb is independently selected from OH, C1-C6 alkoxy, NH2, NH(C1-C6 alkyl) or N(C1-C6 alkyl)2 ;
- R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
- R13 and R14 are independently selected from H, C1-C8 alkyl and C1-C8 alkyl aryl;
- Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, -(CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
- R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
- R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
- R8 is selected from H, OR, NHR15, NR15R16, NH-NHR13, SH, CN, N3 and halogen; wherein R15 and R16 are independently selected from H, C1-C8 alkyl and C1-C8 alkyl aryl; and - CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
- Y is selected from CH, CH2, C(CH3)2 and CCH3;
- 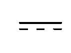 represents a single or double bond according to Y; and
- 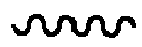 represents the alpha or beta anomer depending on the position of R1, or a compound of formula (Ia) 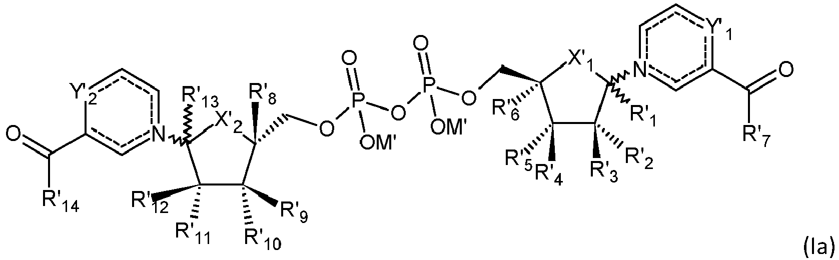 or pharmaceutically acceptable salts, hydrates, crystals, stereoisomers and/or solvates thereof, wherein:
- X'1 and X'2 are independently selected from O, CH2, S, Se, CHF, CF2 and C=CH2;
- R'1 and R'13 are independently selected from H, azido, cyano, C1-C8 alkyl, C1-C8 thio-alkyl, C1-C8 heteroalkyl and OR; wherein R is selected from H and C1-C8 alkyl;
- R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
- R'6 and R'8 are independently selected from H, azido, cyano, C1-C8 alkyl and OR; wherein R is selected from H and C1-C8 alkyl;
- R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
- Y'1 and Y'2 are independently selected from CH, CH2, C(CH3)2 or CCH3;
- M' is selected from H or a suitable counterion;
- 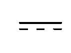 represents a single or a double bound depending on Y'1 and Y'2; and
- 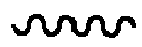 represents the alpha or beta anomer depending on the position of R'1 and R'13, and their combinations and at least one carrier for use in the improvement of sport performance.

2. Composition for use according to claim 1 wherein the improvement of sport performance is chosen amongst the improvement of maximal aerobic capacity VO2max, the improvement of the maximum heart rate HRmax, the improvement of the ventilatory threshold VT1, the improvement of the ventilatory threshold VT2, the improvement of the physical recuperation after exercise, the improvement of muscular power and their combinations.

3. Composition for use according to any of the preceding claims comprising at least a further ingredient.

4. Composition for use according to claim 3 where the at least further ingredient selected amongst amino-acids, omega 3 fatty acids, vitamins or minerals and their combinations.

5. Composition for use according to claim 4 wherein the amino-acid is chosen amongst alanine, arginine, aspartic aid, asparagine, cysteine, glutamic acid, glutamine, glycine, histidine, isoleucine, leucine, lysine, methionine, phenylalanine, proline, serine, threonine, tryptophan, tyrosine, valine and their combinations.

6. Composition for use according to claim 5 comprising at least one of Leucine, Isoleucine, Valine and their combination.

7. Composition for use according to claim 5 comprising at least one of Leucine, Isoleucine, Valine and their combination and/or at least one of Arginine, Glutamine and their combination.

8. Composition for use according to claim 7 comprising Leucine, Isoleucine, Valine, Arginine, and Glutamine.

9. Composition for use according to any of claims 4 - 8 wherein the vitamin is chosen amongst vitamin B1, vitamin B2, vitamin B3, vitamin B5, vitamin B6, vitamin B2, vitamin B8, vitamin B9, vitamin B12, vitamin C, vitamin E and their combinations, preferably vitamin B3, vitamin C and their combination.

10. Composition for use according to any of claims 4-9 wherein the mineral is chosen amongst calcium, phosphate, magnesium, iron, zinc, chromium, boron, vanadium, selenium and their combinations, preferably chromium, iron, magnesium and their combinations.

11. Composition for use according to any of claims 4-10 wherein the omega 3 fatty acids is alpha-linolenic acid (ALA), eicosapentaenoic acid (EPA), docosahexaenoic acid (DHA) and their combinations.

12. Composition for use according to any of the preceding claims wherein compound of formula (I) is chosen amongst dihydro-nicotinamide mononucleotide, compounds IA, compound IB, compound IC, compound ID, compound IE, compound IF, compound IG, compound, compound IH, compound I-J as shown in table 1, preferably I-B, I-D or IF.

13. Composition for use according to any of the preceding claims wherein compound of formula (Ia) is chosen amongst compounds la-A, compound la-B, compound la-C, compound la-D, compound la-E, compound la-F, compound la-G, compound la-H, compound Ia-I, as shown in table 2.

14. Kit of parts comprising a multi-day supply of unit dosages of the composition according to the invention and instructions directing the administration of said multi-day supply over a period of multiple days.
